# EUROPÄISCHE PATENTSCHRIFT

(11) **EP 2 359 136 B1**
(45) Veröffentlichungstag und Bekanntmachung des Hinweises auf die Patenterteilung: **23.01.2019**
(21) Anmeldenummer: 09753073.7
(22) Anmeldetag: 06.11.2009
(51) Int. Cl.: B01L 3/00, G01N 33/552, G01N 33/52, G01N 21/84

(54) **FEINKÖRNIGE FÜLLSTOFFE FÜR PHOTOMETRISCHE REAKTIONSFILME**
FINE-GRAINED FILLER SUBSTANCES FOR PHOTOMETRIC REACTION FILMS
CHARGES EN GRAINS FINS POUR FILM RÉACTIF DE PHOTOMÉTRIE

(30) Priorität: 07.11.2008 EP 08168666
(43) Veröffentlichungstag der Anmeldung: 24.08.2011
(73) Patentinhaber: Roche Diabetes Care GmbH, 68305 Mannheim (DE); F. Hoffmann-La Roche AG, 4070 Basel (CH)
(72) Erfinder: KNAPPE, Wolfgang-Reinhold, 67071 Ludwigshafen (DE); HILLER, Bernd, 68623 Lampertheim (DE); LEHR, Ursula, 76829 Landau (DE); ZIMMER, Volker, 54497 Morbach (DE); PETRICH, Wolfgang, 76669 Bad Schönborn (DE); BEDON-GOMEZ, Luis, David, C11. 71 2a-28 (CO)
(74) Vertreter: Herzog, Fiesser & Partner Patentanwälte PartG mbB
(86) Internationale Anmeldenummer: PCT/EP2009/064757
(87) Internationale Veröffentlichungsnummer: WO 2010/052306

(56) Entgegenhaltungen:
- WO-A1-2006/065900
- WO-A2-2004/085995
- DE-A1- 3 332 144
- US-A- 4 390 343
- US-A- 5 260 195
- US-A- 5 968 765
- US-A1- 2004 077 103
- US-B1- 6 232 066

## Beschreibung

### Gebiet der Erfindung

Die Erfindung betrifft ein diagnostisches Testelement zum Nachweis eines Analyten in einer Probe einer Körperflüssigkeit und ein Verfahren zur Herstellung eines derartigen diagnostischen Testelements. Derartige diagnostische Testelemente werden beispielsweise zum Nachweis eines oder mehrerer Analyten in Körperflüssigkeiten wie Vollblut eingesetzt, beispielsweise zum Nachweis von Glucose, Harnsäure, Ethanol oder Lactat oder ähnlichen Analyten. Auch andere Anwendungen sind jedoch möglich.

### Stand der Technik

Aus dem Stand der Technik sind zahlreiche diagnostische Testelemente bekannt, welche zum Nachweis mindestens eines Analyten in einer Probe einer Körperflüssigkeit eingesetzt werden können. Bei dem mindestens einen Analyten kann es sich beispielsweise um einen Metaboliten handeln. Es können ein qualitativer und/oder auch ein quantitativer Nachweis des Analyten erfolgen. Bekannte Analyte sind beispielsweise Glucose, insbesondere Blutglucose, Harnsäure, Ethanol und/oder Lactat. Alternativ oder zusätzlich sind auch andere Arten von Analyten nachweisbar. Bei der Körperflüssigkeit kann es sich beispielsweise um Vollblut, Blutplasma, intestitielle Flüssigkeit, Speichel, Urin oder andere Arten von Körperflüssigkeiten handeln. Die Erfindung wird im Folgenden, ohne Beschränkung weiterer möglicher Ausgestaltungen, im Wesentlichen unter Bezugnahme auf den Nachweis von Glucose in Vollblut, beschrieben.

Testelemente weisen in der Regel mindestens ein Nachweisreagens zum qualitativen und/oder quantitativen Nachweis des Analyten auf. Unter einem Nachweisreagens ist dabei allgemein eine chemische Substanz oder ein chemisches Substanzgemisch zu verstehen, welches bei Anwesenheit des mindestens einen Analyten mindestens eine nachweisbare Eigenschaft ändert, insbesondere eine physikalisch und/oder chemisch nachweisbare Eigenschaft. Vorzugsweise findet diese Eigenschaftsänderung spezifisch ausschließlich bei Anwesenheit des mindestens einen nachzuweisenden Analyten statt, nicht hingegen bei Gegenwart anderer Substanzen. Es kann jedoch in der Praxis eine unspezifische Eigenschaftsänderung in einem gewissen Rahmen toleriert werden, bei Anwesenheit anderer chemischer Substanzen, deren Anwesenheit in der Probe der Körperflüssigkeit in der Regel unwahrscheinlich ist und/oder welche in lediglich sehr geringer Konzentration vorhanden sind.

Bei der mindestens einen Eigenschaftsänderung kann es sich beispielsweise um die Änderung einer optisch nachweisbaren Eigenschaft handeln, insbesondere eine Farbänderung. Beispiele diagnostischer Testelemente mit optischen Nachweisreagenzien sind aus dem Stand der Technik hinlänglich bekannt. Beispielsweise beschreibt EP 0 821 234 B1, auf welche im Rahmen der vorliegenden Erfindung in weiten Teilen Bezug genommen werden kann, einen diagnostischen Testträger zur Bestimmung eines Analyten aus Vollblut mit Hilfe eines in dem Träger enthaltenen Reagenzsystems, welches ein Farbbildungsreagenz einschließt. Der diagnostische Testträger umfasst ein Testfeld, welches eine Probenaufgabeseite, auf die die Probe zugeführt wird, und eine Nachweisseite, auf der in Folge der Reaktion des Analyten mit dem Reagenzsystem eine optisch nachweisbare Veränderung stattfindet, aufweist. Das Testfeld ist derart ausgestaltet, dass die in der Probe enthaltenen Erythrozyten nicht auf die Nachweisseite gelangen. Das Testfeld weist weiterhin eine transparente Folie und eine erste Filmschicht sowie eine zweite, auf die erste Filmschicht aufgebrachte Filmschicht auf. Die sich auf der transparenten Folie befindliche erste Schicht ist im feuchten Zustand dabei wesentlich weniger lichtstreuend als die darüber liegende zweite Schicht. Die erste Filmschicht umfasst einen Füllstoff, dessen Brechungsindex nahe beim Brechungsindex von Wasser liegt, wohingegen die zweite Schicht ein Pigment mit einem Brechungsindex von bevorzugt mindestens oder sogar > 2,0, insbesondere von mindestens 2,2, in einer Konzentration von bevorzugt mindestens 25 Gew.-% oder sogar mehr als 25 Gew.-%, bezogen auf die getrocknete zweite Schicht, enthält. Beispielsweise kann die erste Schicht als Füllstoff ein Natrium-Aluminium-Silikat umfassen.

In US 4,312,834 wird ein diagnostisches Agens für die Detektion einer Materialkomponente offenbart. Dabei wird ein wasserunlöslicher Film offenbart, welcher aus einem Filmbildner und einem Filmöffner in Form feiner, unlöslicher anorganischer oder organischer Partikel zusammengesetzt ist. Der Filmöffner dient dem Zweck, den Film porös auszugestalten, so dass eine hinreichende Probenaufnahme durch den Film erfolgen kann. Dementsprechend wird beispielsweise vorgeschlagen, Pigmente, also Partikel einer hohen Teilchengröße, beispielsweise Titandioxid-Pigmente, als Filmöffner zu verwenden.

In WO 2006/065900 A1 wird ein Teststreifen oder elektrochemischer Sensor zur Messung der Menge eines Analyten in einer biologischen Flüssigkeit beschrieben. Dieser umfasst ein Enzymsystem für die Reaktion mit dem Analyten. Das reaktive System ist in eine wasserlösliche, quellbare Polymermatrix eingemischt, die kleine, Wasser-unlösliche Partikel mit einer nominellen Größe von ungefähr 0,05 bis 20 Mikrometern umfasst. Es wird somit eine reduzierte Porosität unter Verwendung kleiner Teilchen beschrieben.

In DE 33 32 144 A1 wird ein Element zur analytischen Bestimmung eines speziellen Bestandteils in einer flüssigen Probe, das aufeinanderfolgende wesentliche Schichten in Form einer Rezeptorschicht und einer Verteilerschicht auf einen Träger aufweist. Ein solches Element enthält ein Elektronenübertragungsmittel, eine Farbbildnervorstufe, ein Coenzym vom Oxidationstyp und ein Reagenz, das zur Umwandlung des Coenzyms vom Oxidationstyps mittels des speziellen Bestandteils imstande ist.

Die aus dem Stand der Technik bekannten Testelemente, insbesondere Testelemente mit mindestens einem Testfeld, weisen jedoch in der Praxis Nachteile und technische Herausforderungen auf. So hat es sich gezeigt, dass übliche Testfelder, wie sie aus dem Stand der Technik bekannt sind, zu einer körnigen, inhomogenen Farbentwicklung führen können. Eine derartige inhomogene Farbentwicklung ist für herkömmliche analytische Testgeräte in der Regel jedoch irrelevant, da diese vergleichsweise große Messflecken aufweisen. So weisen beispielsweise handelsübliche optische Blutglucose-Messgeräte Messflecken von ca. 1,5 mm Durchmesser auf. Bei derartigen Durchmessern liegt beispielsweise ein mittlerer Variationskoeffizient, also das Verhältnis aus einer Standardabweichung und einem Mittelwert der Messung, über einen Messbereich von typischerweise ca. 10 bis 600 mg pro dl Blutglucose, typischerweise um ca. 1,5 %.

Gerätetechnisch ist jedoch ein Trend hin zu analytischen Messgeräten mit kleineren Messflecken zu verzeichnen. Bei kleineren Messflecken machen sich jedoch Inhomogenitäten, insbesondere bei der Farbentwicklung der Nachweisreaktion, stärker bemerkbar. So steigt beispielsweise bei Messflecken mit einem Durchmesser von unterhalb von 0,5 mm der Variationskoeffizient deutlich an, insbesondere über den klinisch noch akzeptablen Wert von ca. 4 %. Da die Entwicklung integrierter Blutglucose-Messsysteme zu immer kleineren Blutvolumina, bis hin zu ca. 100 Nanolitern, führt, müssen hier Messflecke in der Grö ßenordnung von 10 Mikrometern x 10 Mikrometern, insbesondere bei Verwendung ortsaufgelöster Optiken, ermöglicht werden. Die bekannten Testfelder weisen jedoch zu diesem Zweck in der Regel nicht die erforderliche Präzision auf.

### Aufgabe der Erfindung

Es ist daher eine Aufgabe der vorliegenden Erfindung, ein diagnostisches Testelement, ein Verfahren zur Herstellung eines diagnostischen Testelements sowie ein Verfahren zum

Nachweis eines Analyten in einer Probe einer Körperflüssigkeit bereitzustellen, welche die Nachteile bekannter diagnostischer Testelemente und bekannter Verfahren zumindest weitgehend vermeiden. Insbesondere soll ein hochpräziser quantitativer Nachweis des mindestens einen Analyten auch in kleinsten Flüssigkeitsmengen ermöglicht werden.

### Offenbarung der Erfindung

Diese Aufgabe wird gelöst durch die Erfindung mit den Merkmalen der unabhängigen Patentansprüche. Vorteilhafte Weiterbindungen der Erfindung, welche einzeln oder in beliebiger Kombination realisierbar sind, sind in den abhängigen Patentansprüchen dargestellt. Dabei werden ein diagnostisches Testelement zum Nachweis eines Analyten in einer Probe einer Körperflüssigkeit, ein Verfahren zur Herstellung eines diagnostischen Testelements zum Nachweis eines Analyten in einer Probe einer Körperflüssigkeit sowie ein Verfahren zum Nachweis eines Analyten in einer Probe einer Körperflüssigkeit vorgeschlagen. Dabei kann das diagnostische Testelement in einer oder mehreren der vorgeschlagenen Ausgestaltungen nach einem erfindungsgemäßen Verfahren herstellbar sein, und das Herstellungsverfahren kann eingesetzt werden, um ein diagnostisches Testelement in einer oder mehreren der beschriebenen Ausgestaltungen herzustellen. Dementsprechend kann für die Beschreibung möglicher Ausgestaltungen des diagnostischen Testelements auf die Beschreibung des Herstellungsverfahrens verwiesen werden oder umgekehrt. Es sind jedoch grundsätzlich auch andere Ausgestaltungen möglich. Das vorgeschlagene Verfahren zum Nachweis eines Analyten einer Probe einer Körperflüssigkeit wird unter Verwendung eines diagnostischen Testelements in einer oder mehreren der im Folgenden beschriebenen Ausgestaltungen durchgeführt.

In einem ersten Aspekt der vorliegenden Erfindung wird somit ein diagnostisches Testelement zum Nachweis eines Analyten in einer Probe einer Körperflüssigkeit vorgeschlagen. Für die möglichen Ausgestaltungen dieses Testelements kann grundsätzlich auf die obige Beschreibung des Standes der Technik verwiesen werden. Der mindestens eine Analyt kann somit beispielsweise quantitativ oder qualitativ nachgewiesen werden. Bei dem mindestens einen Analyten kann es sich insbesondere um einen oder mehrere der Analyte Glucose, Harnsäure, Ethanol, Lactat oder um eine Kombination dieser Analyte und/oder anderer Analyte handeln. Auch andere Analyte sind jedoch grundsätzlich nachweisbar, beispielsweise einer oder mehrere der oben genannten Analyte. Bei der Probe der Körperflüssigkeit kann es sich insbesondere um Vollblut handeln. Auch andere Ausgestaltungen sind jedoch grundsätzlich möglich, wobei auf die obige Beschreibung verwiesen werden kann.

Das diagnostische Testelement umfasst mindestens ein Testfeld mit mindestens einem Nachweisreagens. Unter einem Testfeld ist dabei eine zusammenhängende Fläche des Nachweisreagens zu verstehen, insbesondere ein Film mit einer oder mehreren Schichten, welcher, wie unten noch näher erläutert wird, beispielsweise auf mindestens ein Trägerelement aufgebracht sein kann. Das Nachweisreagens ist eingerichtet, um bei Anwesenheit des Analyten mindestens eine nachweisbare Veränderung durchzuführen. Insbesondere kann es sich bei dieser nachweisbaren Veränderung um eine physikalisch und/oder chemisch nachweisbare Veränderung handeln. Im Folgenden wird insbesondere Bezug genommen auf physikalische Änderungen in Form einer optisch nachweisbaren Veränderung, insbesondere einer Farbänderung. Grundsätzlich sind jedoch auch andere Arten von nachweisbaren Veränderungen alternativ oder zusätzlich denkbar, beispielsweise chemisch und/oder elektrochemisch nachweisbare Veränderungen. Das Nachweisreagens kann insbesondere mindestens ein Enzym umfassen, beispielsweise Glucosedehydrogenase (beispielsweise FAD-, NAD⁺- oder PQQ-abhängig) und/oder Glucoseoxydase. Allgemein lassen sich somit beispielsweise Glucose-Nachweise einsetzen, welche eine oder mehrere der folgenden enzymatischen Nachweise oder Nachweisreagenzien umfassen: GOD, GlucDOR (PQQ-abhängige GDH und ihre Mutanten), FAD-GDH, NAD-abhängige GDH mit Mediator (beispielsweise Diaphorase) zur Übertragung der Redoxäquivalente von NADH auf einen Nitrosoanilin-Mediator.

Weiterhin kann das Nachweisreagens, alternativ oder zusätzlich, einen oder mehrere Mediatoren umfassen, also Stoffe, welche elektrische Ladungen von einem Stoff auf einen anderen übertragen können. Insbesondere können Mediatoren eingesetzt werden, welche für einen Elektronenübertrag geeignet sind. Beispielsweise kann es sich bei diesem Stoff um Nitrosoanilin handeln. Weiterhin kann, wiederum alternativ oder zusätzlich, das Nachweisreagens mindestens einen Indikator umfassen. Unter einem Indikator kann eine Substanz verstanden werden, welche als solche mindestens eine Eigenschaft ändern kann, die nachgewiesen werden kann, je nachdem, in welcher Form diese Substanz vorliegt. Beispielsweise können Substanzen eingesetzt werden, welche in einer oxidierten und einer reduzierten Form unterschiedliche optische Eigenschaften, beispielsweise unterschiedliche Farben, aufweisen können. Alternativ oder zusätzlich kann der Indikator eine Substanz umfassen, welche in unterschiedlichen Ladungszuständen unterschiedliche optische Eigenschaften aufweist, beispielsweise unterschiedliche Farbeigenschaften. Allgemein kann also unter einem Nachweisreagens eine einzelne Substanz oder ein Substanzgemisch, beispielsweise, wie oben ausgeführt, ein Gemisch aus mindestens einem Enzym, mindestens einem Mediator und mindestens einem Indikator, verstanden werden. Derartige Nachweisreagenzien sind aus dem Stand der Technik grundsätzlich bekannt, beispielsweise aus dem oben beschriebenen Stand der Technik.

Das Testfeld weist mindestens eine das Nachweisreagens umfassende Nachweisschicht auf. Dabei kann ein System mit einer einzigen Nachweisschicht verwendet werden, oder es können mehrere Nachweisschichten eingesetzt werden, welche direkt oder unter Zwischenschaltung einer oder mehrerer weiterer Schichten aufeinander aufgebracht werden können. Besonders bevorzugt ist jedoch ein System mit lediglich einer einzigen Nachweisschicht. Unter einer Schicht ist dabei im Rahmen der vorliegenden Erfindung allgemein ein Element zu verstehen, bei welchem ein Schichtmaterial flächig auf ein Trägerelement aufgebracht wird oder als freistehender Film geformt wird. Die Schicht kann, muss jedoch nicht notwendigerweise geschlossen ausgestaltet sein, sondern kann beispielsweise auch Öffnungen aufweisen. Besonders bevorzugt ist jedoch, wie unten noch näher ausgeführt wird, eine im Wesentlichen gleichförmige, vorzugsweise lochfreie, homogene Ausgestaltung der Nachweisschicht mit einer homogenen Schichtdicke. Die Schichtdicke, also die mittlere Dicke der Nachweisschicht, liegt dabei vorzugsweise bei 3 bis 60 Mikrometern, insbesondere bei 5 bis 15 Mikrometern, beispielsweise bei 8 Mikrometern.

Die Nachweisschicht weist Partikel auf. Unter Partikeln sind allgemein im Rahmen der vorliegenden Erfindung Festkörper im Mikrometer-Bereich oder Nanometer-Bereich zu verstehen, welche nicht unmittelbar untereinander verbunden sind und welche somit im trockenen Zustand und ohne sonstige Stoffe der Nachweisschicht beispielsweise ein rieselfähiges Pulver bilden können. Partikel können beispielsweise allgemein feste Bestandteile von Erosolen, Suspensionen oder Pulvern bilden.

Erfindungsgemäß wird vorgeschlagen, dass die Partikel eine Partikelgrößenverteilung aufweisen, insbesondere im trockenen Zustand der Nachweisschicht, bei welcher mindestens 90 % sämtlicher Partikel der Nachweisschicht eine tatsächliche Partikelgröße von weniger als 10 Mikrometern, vorzugsweise von weniger als 3 Mikrometern oder sogar weniger als 1 Mikrometer, aufweisen.

Unter der Nachweisschicht, für welche diese Bedingung gelten soll, ist dabei die gesamte Nachweisschicht, deren Veränderung messbar ist, zu verstehen. Insbesondere kann es sich dabei, wenn eine optisch nachweisbare Veränderung wie beispielsweise eine Farbänderung gemessen wird, um die gesamte optisch erkennbare Nachweisschicht handeln, optional bis hin zu einer Reflexionsschicht oder Abtrennschicht, welche auf einer Probenaufgabeseite, wie unten noch näher erläutert wird, auf die Nachweisschicht aufgebracht wird. So kann die Nachweisschicht beispielsweise, wie unten noch erläutert wird, von mindestens einer weiteren Schicht überlagert sein, welche beispielsweise reflektive Eigenschaften aufweisen kann. Die Schichten müssen dabei nicht notwendigerweise klar voneinander abgegrenzt sein. Lokal ist dabei, von der Nachweisseite betrachtet, unter der Nachweisschicht jeweils die Schicht zu verstehen, welche vermessen wird, bis hin beispielsweise zu den reflektierenden Partikeln und/oder einem anderen reflektierenden Objekt einer mittelbar oder unmittelbar angrenzenden Schicht.

Unter einer Partikelgröße ist dabei ein Äquivalentdurchmesser eines Partikels zu verstehen, also ein Durchmesser einer Kugel, welche ein gleiches Volumen und/oder eine gleiche Oberfläche wie der Partikel aufweist. Dabei können verschiedene Verfahren zur Bestimmung der Partikelgrößenverteilung verwendet werden. Dabei ist zwischen verschiedenen Fällen zu unterscheiden. Für Rohmaterialien, wie beispielsweise ein oder mehrere Füllstoffe, welche Bestandteile der Nachweisschicht sein können, kann die Partikelgröße beispielsweise mittels einer Laserstreuung und/oder einer Laserbeugung ermittelt werden. In einem Schichtaufbau, beispielsweise der Nachweisschicht und optional einer darauf aufgebrachten Abtrennschicht, können auch optische Verfahren eingesetzt werden, beispielsweise Verfahren, welche auf einer Bilderkennung basieren. Auf diese Weise kann beispielsweise eine Partikelgrößenverteilung, beispielsweise innerhalb der Nachweisschicht, bis hinab in einen Bereich von 3 Mikrometern bis 10 Mikrometern ermittelt werden. Wiederum alternativ oder zusätzlich können auch andere Verfahren eingesetzt werden, wie beispielsweise eine Rasterelektronenmikroskopie an Proben, beispielsweise Mikrotom-Querschnitten. Mittels derartiger Verfahren können beispielsweise Partikelgrößen und Partikelgrößenverteilungen in der Nachweisschicht und optional auch in einer oder mehreren darauf aufgebrachten Schichten, wie beispielsweise der optionalen Abtrennschicht, ermittelt werden. Auch eine eindeutige Identifizierung der Partikel kann erfolgen, beispielsweise indem zusätzlich eine energiedispersive Röntgenspektroskopie (EDX) eingesetzt wird. Bei Verwendung elektronenmikroskopischer Verfahren reicht die Auflösung typischerweise in den Bereich von Nanometern, wobei beispielsweise sämtliche Partikel mit einer Partikelgröße > 1 Nanometer erfasst werden können. Allgemein sind Vorrichtungen und Verfahren zur Bestimmung der Partikelgrößenverteilung dem Fachmann bekannt und kommerziell erhältlich. Im Rahmen der vorliegenden Erfindung ist, da vorzugsweise die nachweisbare Veränderung eine optisch nachweisbare Veränderung ist, eine optische Bestimmung der Partikelgrößenverteilung einsetzbar. Beispielsweise kann es sich dabei um eine automatische Bildanalyse eines Bildes der Nachweisschicht handeln, wie unten noch näher ausgeführt wird. Diese automatische Bilderkennung kann dabei beispielsweise dadurch erfolgen, dass mittels einer Kamera oder eines anderen ortsauflösenden Bilddetektors ein Bild zumindest eines Teils der Nachweisschicht aufgenommen wird und dann mittels einer Bilderkennung einzelne Partikel erkannt und einer Größenverteilung zugeordnet werden. Dabei können allgemein zur Bestimmung der Partikelgrößenverteilung beispielsweise alle erkannten Partikel verwendet werden. Da jedoch in der Praxis in der Regel Partikel erst ab einer Mindestgröße erst als solche erkannt werden, können beispielsweise auch nur Partikel ab einer vorgegebenen Mindestgröße bei der Bestimmung der Partikelgrößenverteilung berücksichtigt werden, beispielsweise erst ab einer Mindestgröße von 10 Nanometern bis 200 Nanometern, insbesondere einer Partikelgröße von 50 Nanometern bis 100 Nanometern.

Unter einer tatsächlichen Partikelgröße ist dabei im Rahmen der vorliegenden Erfindung die Partikelgröße der Partikel in der Nachweisschicht zu verstehen, in der Form, in welcher die Partikel tatsächlich in der Nachweisschicht vorliegen. Setzen sich die Partikel in der Nachweisschicht aus mehreren Primärpartikeln zusammen, beispielsweise in Form von Agglomeraten und/oder Aggregaten, welche zusammenhaften, so ist der Äquivalentdurchmesser des Aggregats bzw. Agglomerats einzusetzen, nicht hingegen der Äquivalentdurchmesser der Primärpartikel. Nicht erfasst von der vorliegenden Erfindung sind also Fälle, in welchen die Nachweisschicht zwar derart hergestellt wird, dass für deren Herstellung Pulver verwendet werden, welche nominell die genannten Eigenschaften aufweisen, bei welchen die Partikel des Pulvers jedoch, beispielsweise während der Herstellung der Nachweisschicht, derart miteinander wechselwirken, dass in der fertigen und vorzugsweise trockenen Nachweisschicht Agglomerate und/oder Aggregate vorliegen, so dass insgesamt für alle Partikel in der fertigen Nachweisschicht die genannte Bedingung nicht mehr erfüllt ist.

Insbesondere können mindestens 80 % sämtlicher Partikel der Nachweisschicht eine tatsächliche Partikelgröße von weniger als 5 Mikrometern, insbesondere von weniger als 1 Mikrometer, aufweisen. Mindestens 70 % sämtlicher Partikel der Nachweisschicht weisen eine tatsächliche Partikelgröße von weniger als 900 Nanometern, vorzugsweise von weniger als 800 Nanometern, auf.

Im Rahmen der vorliegenden Beschreibung können die Partikel der Nachweisschicht dabei insbesondere eine mittlere Partikelgröße von 10 Nanometern bis 5 Mikrometern aufweisen, vorzugsweise von weniger als 1 Mikrometer. Vorzugsweise kann die mittlere Partikelgröße von 20 Nanometern bis 1 Mikrometer und besonders bevorzugt von 20 Nanometern bis 500 Nanometern sein. Alternativ oder zusätzlich kann vorzugsweise die mittlere Partikelgröße von 70 Nanometern bis 5 Mikrometern, insbesondere von 70 Nanometern bis 1 Mikrometer und besonders bevorzugt von 70 Nanometern bis 500 Nanometern sein.

Im Rahmen der vorliegenden Beschreibung können können die Partikel der Nachweisschicht insbesondere eine mittlere Partikelgröße von weniger als 1 Mikrometer, insbesondere von weniger als 500 Nanometern und besonders bevorzugt von maximal 300 Nanometern, oder sogar weniger als 100 Nanometern, beispielsweise 25 Nanometern oder weniger, aufweisen.

Unter einer mittleren Partikelgröße kann dabei beispielsweise ein Median sämtlicher Partikelgrößen der Partikelgrößenverteilung verstanden werden, welcher üblicherweise als d₅₀ bezeichnet wird. Dieser Median ist derart gewählt, dass etwa 50 % der Partikel eine Partikelgröße unterhalb des d₅₀-Werts aufweisen, und etwa 50 % der Partikel eine Partikelgröße oberhalb dieses Medians.

Die Partikel können insbesondere eines oder mehrere der folgenden Materialien umfassen: SiO₂; Kieselgur; ein Silikat, insbesondere ein Natrium-Aluminium-Silikat; ein Metalloxid, insbesondere ein Aluminiumoxid und/oder ein Titanoxid; ein synthetisches oxidisches Material, insbesondere ein nanoteiliges oxidisches Material, insbesondere ein nanoteiliges Siliziumoxid und/oder Aluminiumoxid und/oder Titanoxid; Kaolin; Pulverglas; Fällungskieselsäure; Calciumsulfat x 2 H₂O.

Besonders bevorzugt ist es, wenn sämtliche Partikel der Nachweisschicht mit einer Partikelgröße von mehr als 10 Nanometern, insbesondere von mehr als 20 Nanometern oder von mehr als 100 Nanometern anorganische Partikel sind. Wie oben bereits definiert, soll vom Begriff "Partikel" ein organischer Filmbildner und ein daraus gebildeter organischer Film nicht umfasst sein, da Filme allgemein nicht aus losen Teilchen zusammengesetzt sind, welche untereinander nicht verbunden sind, sondern da Filme in der Regel eine kontinuierliche Schicht bilden. Die Partikel der Nachweisschicht können jedoch, wie unten noch näher ausgeführt wird, in mindestens einen derartigen Filmbildner eingebettet sein.

Die Nachweisschicht kann insbesondere einen Brechungsindex von 1,1 bis 1,8, vorzugsweise von 1,2 bis 1,5, aufweisen. Damit kann die Nachweisschicht insbesondere, sei es in trockenem Zustand oder auch im feuchten Zustand, einen Brechungsindex aufweisen, welcher nahe dem Brechungsindex von Wasser (ca. 1,33) liegt.

Das diagnostische Testelement, insbesondere das mindestens eine Nachweisreagens und/oder die mindestens eine Nachweisschicht, kann insbesondere derart eingerichtet sein, dass die nachweisbare Veränderung innerhalb einer Zeitdauer abgeschlossen ist, welche weniger als 60 Sekunden, vorzugsweise weniger als 40 Sekunden und besonders bevorzugt 20 Sekunden oder weniger beträgt. Diese Zeitdauer kann auch als Reaktionszeit bezeichnet werden. Beinhaltet beispielsweise die nachweisbare Veränderung eine optische Veränderung in Form einer Farbänderung, so kann als Reaktionszeit beispielsweise diejenige Zeitspanne ab dem Aufbringen der Probe auf das Testfeld definiert werden, innerhalb derer eine Farbreaktion soweit abgeschlossen ist, dass sich die Relativremission anschließend um weniger als 1 % pro halber Sekunde ändert. Die Relativremission kann beispielsweise das Verhältnis der Remission zu einer Remission eines Testelements ohne Probe und/oder zu einem Kalibrationsstandard sein. Die Reaktionszeit kann beispielsweise durch eine entsprechende Auswahl der Testchemie des Nachweisreagens und/oder durch die gesamte Zusammensetzung des Testfeldes und/oder gerade durch die im Rahmen der vorliegenden Erfindung verwendete Partikelgrößenverteilung eingestellt werden.

Das Testfeld kann insbesondere eine Aufgabenseite zum Aufbringen der Probe der Körperflüssigkeit und eine Nachweisseite zum Nachweisen einer Veränderung des Nachweisreagenz, insbesondere einer optischen Veränderung, beispielsweise einer Farbänderung, aufweisen. Das Testfeld weist weiterhin mindestens eine Abtrennschicht auf. Diese Abtrennschicht kann mehrere Funktionen aufweisen. So kann diese beispielsweise zum Abtrennen grober Bestandteile der Probe eingerichtet sein, insbesondere zum Abtrennen von Erythrozyten. Alternativ oder zusätzlich kann die Abtrennschicht auch eingerichtet sein, um eine Eigenfarbe der Probe zu überdecken, beispielsweise eine Bluteigenfarbe. Zu diesem Zweck kann, wie unten noch näher ausgeführt wird, die Abtrennschicht beispielsweise mindestens ein Pigment umfassen, vorzugsweise mindestens ein weißes Pigment. Wiederum alternativ oder zusätzlich kann die Abtrennschicht auch eingerichtet sein, um eine Reflexionsfunktion zu erfüllen, beispielsweise um ein Messlicht, welches in die Nachweisschicht eingestreut wird, und/oder in der Nachweisschicht emittiertes Licht wie beispielsweise Fluoreszenzlicht zu reflektieren.

Die Abtrennschicht ist auf einer der Aufgabenseite zuweisenden Seite der Nachweisschicht angeordnet. Beispielsweise kann die Abtrennschicht direkt oder indirekt auf die Nachweisschicht aufgebracht sein. Unter einem indirekten Aufbringen kann dabei beispielsweise die Zwischenschaltung einer oder mehrerer weiterer Schichten verstanden werden. Die Abtrennschicht kann insbesondere derart eingerichtet sein, dass grobe Bestandteile der Probe, insbesondere bei Vollblut-Erythrozyten, nicht zur Nachweisseite der Nachweisschicht oder überhaupt nicht zur Nachweisschicht gelangen können.

Unter groben Bestandteilen können dabei allgemein Bestandteile verstanden werden, welche eine
Größe, beispielsweise eine Partikelgröße und/oder einen Äquivalentdurchmesser, von mehr als 1 Mikrometer aufweisen, insbesondere von mehr als 5 Mikrometern. Insbesondere Erythrozyten, welche eine charakteristische und intensive eigene Farbe aufweisen, können den üblichen Farbnachweis von Blutglucose, beispielsweise mittels der oben beschriebenen Nachweisreagenzien, auf der Nachweisseite stören oder sogar verhindern.

Die Abtrennschicht kann insbesondere grobkörnig ausgestaltet sein, d. h. ebenfalls partikulär aufgebaut sein, wobei die Partikel dieser Abtrennschicht gröber ausgestaltet sein können als die Partikel der Nachweisschicht. Insbesondere kann die Abtrennschicht Partikel von mehr als einem Mikrometer Partikelgröße aufweisen. Die Abtrennschicht weist mindestens ein Pigment auf, also einen partikulären Farbstoff, vorzugsweise einen anorganischen Farbstoff, mit Partikeln, die eine mittlere Partikelgröße aufweisen, welche oberhalb der für einen optischen Nachweis verwendeten Lichtwellenlänge liegt, beispielsweise oberhalb einer Wellenlänge von 660 Nanometern. Insbesondere kann die Abtrennschicht, wie oben ausgeführt, mindestens ein Pigment zur optischen Überdeckung einer Eigenfarbe von Blut aufweisen. Die Abtrennschicht kann insbesondere mindestens ein weißes Pigment (Weißpigment) umfassen. Die Abtrennschicht kann beispielsweise eines oder mehrere der folgenden Pigmente umfassen: Titandioxid; Zirkondioxid; Bariumtitanat; Bariumzirkonat; Zirkoniumsilikat. Auch eine Kombination der genannten und/oder anderer Pigmente ist möglich. Besonders bevorzugt ist die Verwendung von Zirkondioxid und/oder Titandioxid. das Pigment hat vorzugsweise zur optimalen Reflexion des Lichts eine mittlere Partikelgröße zwischen 200 Nanometern und 400 Nanometern.

Alternativ oder zusätzlich kann die Abtrennschicht optional mindestens einen Füllstoff aufweisen, vorzugsweise einen Füllstoff mit einem Brechungsindex < 2,0. Mittels dieses Füllstoff können beispielsweise ein Saugverhalten und/oder eine Lichtdurchlässigkeit der Abtrennschicht eingestellt werden. Der mindestens einen Füllstoff kann beispielsweise Kieselsäure und/oder ein Silikat umfassen. Beispielsweise kann der Füllstoff eine mittlere Partikelgröße < 5 Mikrometern aufweisen.

Insbesondere kann die Abtrennschicht ein Pigment mit einem Brechungsindex von mindestens 2,0, vorzugsweise von mindestens 2,2 oder sogar mindestens 2,5, in einer Konzentration von mindestens 25 Gew.-%, bezogen auf eine getrocknete Schicht, also eine getrocknete Abtrennschicht, aufweisen. Bei diesem Pigment kann es sich insbesondere um Titandioxid-Partikel und/oder Zirkondioxid-Partikel handeln, oder das Pigment kann diese Arten von Partikeln umfassen. Auch andere Ausgestaltungen sind jedoch möglich. Die Titandioxid-Partikel bzw. Zirkondioxid-Partikel können insbesondere eine mittlere Partikelgröße von beispielsweise zumindest näherungsweise 300 Nanometern aufweisen. Abweichungen von vorzugsweise nicht mehr als 50%, besonders bevorzugt von nicht mehr als 10%, können dabei jedoch tolerierbar sein. Eine Partikelgröße von 300 Nanometern ist in der Regel optimal für ein sichtbares Licht reflektierendes Weißpigment.Titandioxid-Partikel weisen besonders lichtstreuende Eigenschaften auf, sodass die Abtrennschicht gleichzeitig auch als Reflektionsschicht wirken kann, um von der Nachweisseite eingestrahltes Licht zu remittieren oder zu reflektieren. Alternativ oder zusätzlich kann der Schichtaufbau des Testfeldes jedoch auch weiterhin mindestens eine Reflektionsschicht umfassen, welche die genannten Eigenschaften besitzen kann.

Das diagnostische Testelement kann, wie oben ausgeführt, als Schichtaufbau ausgestaltet sein und/oder kann einen Schichtaufbau umfassen. Es kann neben der mindestens einen Nachweisschicht weiterhin die mindestens eine Abtrennschicht und/oder mindestens eine Reflexionsschicht umfassen. Das Testfeld kann mit seiner Nachweisseite auf mindestens ein Trägerelement aufgebracht sein. Insbesondere kann das diagnostische Testelement somit mindestens ein Trägerelement umfassen, wobei das Trägerelement vorzugsweise mindestens einen transparenten Bereich aufweist. Das Testfeld kann mit seiner Nachweisseite auf den transparenten Bereich aufgebracht sein. Das Trägerelement kann beispielsweise ein flächiges Trägerelement sein, insbesondere ein streifenförmiges Trägerelement. Beispielsweise kann das Trägerelement eine Kunststoffschicht, eine Papierschicht, eine keramische Schicht oder einen Laminataufbau und/oder eine Kombination der genannten Schichten umfassen. Beispielsweise kann das Trägerelement außerhalb des transparenten Bereichs im Wesentlichen opak ausgestaltet sein, sodass lediglich durch den transparenten Bereich hindurch die Nachweisseite des Testfeldes erkennbar ist. Die Aufgabenseite der Probe kann dann auf einer von dem Trägerelement abgewandten Seite des Testfeldes angeordnet sein. Das diagnostische Testelement kann derart ausgestaltet sein, dass die Probe der Körperflüssigkeit unmittelbar auf die Aufgabenseite aufgebracht ist, sodass beispielsweise die Aufgabenseite für einen Benutzer des diagnostischen Testelements unmittelbar zugänglich ist, um beispielsweise eine Probe unmittelbar auf die Fläche der Aufgabenseite, welche zumindest teilweise zugänglich ist, aufzutropfen, aufzutupfen oder auf andere Weise aufzubringen. Alternativ könnte auch ein Transportsystem vorgesehen sein, welches eingerichtet ist, um die Probe der Körperflüssigkeit von einer an einem anderen Ort angeordneten Aufgabestelle zu der Aufgabenseite zu transportieren, was jedoch weniger bevorzugt ist.

Die Nachweisschicht kann, wie oben bereits mehrfach erwähnt, neben den Partikeln und den Nachweisreagenz, weitere Substanzen umfassen. Die Partikel sind vorzugsweise nicht identisch mit dem Nachweisreagens oder zumindest nicht vollständig identisch mit dem Nachweisreagenz, wobei es sich, wie oben beschrieben, bei dem Nachweisreagens auch um eine Mischung mehrerer Nachweisreagenzien oder mehrerer Stoffe, die zusammen das Nachweisreagens bilden, handeln kann. Die Nachweisschicht kann beispielsweise analog zu der in EP 0 821 234 B1 beschriebenen ersten Filmschicht des diagnostischen Testträgers ausgestaltet sein, abgesehen von der oben beschriebenen Partikelgrößenverteilung. Somit kann die Nachweisschicht beispielsweise mindestens einen organischen Filmbildner umfassen. Beispielsweise kann dieser mindestens eine Filmbildner eine Polyvinylproprionat-Dispersion umfassen. Alternativ oder zusätzlich können jedoch auch andere Filmbildner eingesetzt werden.

In einem zweiten Aspekt der vorliegenden Erfindung wird ein Verfahren zur Herstellung eines diagnostischen Testelements zum Nachweis eines Analyten in einer Probe einer Körperflüssigkeit vorgeschlagen. Wie oben ausgeführt, kann es sich bei diesem diagnostischen Testelement insbesondere um ein diagnostisches Testelement gemäß einer oder mehreren der oben beschriebenen Ausgestaltungen oder einer oder mehreren der im Folgenden noch beschriebenen Ausführungsbeispiele handeln. Das diagnostische Testelement weist mindestens ein Testfeld mit mindestens einem Nachweisreagens auf. Das Nachweisreagens ist eingerichtet, um bei Anwesenheit des Analyten mindestens eine Veränderung zu durchlaufen, insbesondere eine optische Veränderung. Das Testfeld weist mindestens eine das Nachweisreagens umfassende Nachweisschicht auf. Bei dem Verfahren wird die Nachweisschicht derart erzeugt, dass diese Partikel aufweist, wobei mindestens 90 % sämtlicher Partikel der Nachweisschicht eine tatsächliche Partikelgröße von weniger als 10 Mikrometern, vorzugsweise von weniger als 3 Mikrometern oder sogar von weniger als 1 Mikrometer, aufweisen. Für besonders bevorzugte Partikelgrößenverteilungen kann auf die obige Beschreibung verwiesen werden.

Die Nachweisschicht kann insbesondere mittels mindestens eines nasschemischen Verfahrens erzeugt werden, insbesondere aus einer oder mehreren Dispersionen, vorzugsweise wässrigen Dispersionen. Derartige Schichtbildungsverfahren aus einer oder mehreren Dispersionen sind dem Fachmann grundsätzlich bekannt, wobei exemplarisch wiederum beispielsweise auf den oben genannten Stand der Technik verwiesen werden kann, insbesondere die EP 0 821 234 B1.

Um sicherzustellen, dass in der fertigen Nachweisschicht die genannten Bedingungen der Partikelgrößenverteilung vorliegen, können verschiedene Verfahren eingesetzt werden. Insbesondere kann zum Bereitstellen der Partikel in der Nachweisschicht mindestens ein Pulver verwendet werden, beispielsweise ein Pigmentpulver. Dieses Pulver kann Agglomerate von Primärteilchen aufweisen, welche bereits unmittelbar in dem Ausgangspulver vorliegen können oder welche sich vorübergehend auch erst während des Herstellungsverfahrens bilden können, beispielsweise in der Dispersion. Dabei wird das Pigmentpulver bei dem vorgeschlagenen Herstellungsverfahren jedoch mittels mindestens eines mechanischen Dispergierverfahrens bearbeitet, um die Agglomerate zumindest teilweise aufzubrechen, sodass in der Nachweisschicht die oben genannte Partikelgrößenverteilung vorliegt. Unter einem Dispergierverfahren ist dabei allgemein ein Verfahren zu verstehen, bei welchem das Pulver, beispielsweise das Pigmentpulver, in mindestens einem flüssigen Medium, vorzugsweise einem wässrigen Medium, verteilt wird, ohne dass sich das Pulver in diesem Medium löst, sodass sich eine Dispersion bildet. Der Dispersion können weitere Stoffe beigemengt werden. Unter einem mechanischen Dispergierverfahren ist dabei - im Gegensatz zu chemischen Dispergierverfahren, welche gleichwohl zusätzlich eingesetzt werden können, was jedoch weniger bevorzugt ist - ein Dispergierverfahren zu verstehen, bei welchem die Verteilung des Pulvers in dem Medium mittels einer mechanischen Einwirkung auf die Dispersion aufrechterhalten wird. Diese mechanische Einwirkung kann insbesondere derart erfolgen, dass bei dieser mechanischen Einwirkung hohe Scherkräfte auf die Dispersion und insbesondere das Pulver und die darin enthaltenen Agglomerate, wovon auch Aggregate erfasst sein sollen, wirken, sodass diese zumindest teilweise aufgebrochen werden, sodass kleinere Partikel entstehen, die die oben genannte Partikelgrößenverteilungsbedingung erfüllen.

Insbesondere kann zur Durchführung des mechanischen Dispergierverfahrens ein Dissolver verwendet werden. Unter einem Dissolver ist allgemeine eine Vorrichtung zu verstehen, welche eine Verteilung des Pulvers in einem Medium, insbesondere eine im Wesentlichen homogene Verteilung, aufrechterhalten kann und gleichzeitig hohe Scherkräfte auf die Dispersion ausüben kann. Beispielsweise können diese Scherkräfte mittels zweier oder mehrerer gegeneinander bewegter, eng zueinander beabstandeter Flächen, zwischen denen die Dispersion aufgenommen ist, ausgeübt werden. So sind beispielsweise Dissolver in Form von Scheibenrührern kommerziell erhältlich, in welchen mittels einer Rührscheibe, die eine Drehbewegung versetzt wird, hohe Scherkräfte auf die Dispersion ausgeübt werden, sodass die Agglomerate auseinander gerissen werden. Alternativ oder zusätzlich können Dissolver nach einem Rotor/Stator-Prinzip eingesetzt werden. Mittels derartiger Dissolver kann also eine Dispersion erzeugt oder aufbereitet werden, aus der die Nachweisschicht erzeugt wird, sodass die oben genannte Bedingung der Partikelgrößenverteilung erfüllt ist.

Alternativ oder zusätzlich kann zur Durchführung des mechanischen Dispergierverfahrens, insbesondere zur Dispergierung der Füllstoffe, ein Dreiwalzenwerk (auch Dreiwalzenstuhl genannt) eingesetzt werden. Bei einem derartigen Dreiwalzenwerk werden mindestens drei Walzen oder Zylinder eingesetzt, welche mit unterschiedlicher Geschwindigkeit gegeneinander laufen. Der Abstand zwischen den Walzen oder Zylindern wird dabei in der Regel vergleichsweise klein eingestellt, beispielsweise auf 1 mm bis hinunter in den Nanometer-Bereich.

Zum Bereitstellen der Partikel können einerseits, wie unten noch näher erläutert wird, kommerziell erhältliche Partikel eingesetzt werden, welche die genannte Bedingung für die Partikelgrößenverteilung erfüllen. Alternativ oder zusätzlich können jedoch auch Mahlverfahren verwendet werden. So kann zum Bereitstellen der Partikel beispielsweise mindestens ein Pulver, insbesondere mindestens ein Pigmentpulver, verwendet werden, wobei das Pulver mindestens einem Mahlschritt unterworfen wird. Unter einem Mahlschritt ist dabei ein Verfahren zu verstehen, bei welchem das Pulver in trockenem oder in nassem Zustand durch Einwirkung mechanischer Kräfte zerrieben wird. Verschiedene Mahlverfahren sind dabei bekannt. So kann der mindestens eine Mahlschritt beispielsweise einen Nassmahlschritt, insbesondere in einer Kugelmühle, und/oder einem Trockenmahlschritt, insbesondere in einer Luftstrahlmühle, umfassen. Auch andere Mahlverfahren sind dem Fachmann bekannt und sind kommerziell verfügbar, sodass entsprechende Mühlen ausgewählt werden können, welche auf die Art des Pulvers und/oder die Art der gewünschten Partikelgrößenverteilung angepasst sein können.

Beim Bereitstellen der Partikel kann insbesondere ein Pulver eines synthetischen oxidischen Materials verwendet werden. Derartige synthetische oxidische Materialien sind teilweise bereits, wie unten exemplarisch noch näher ausgeführt wird, kommerziell in den genannten Partikelgrößen verfügbar, beispielsweise von Materialherstellern, welche sich auf Mikro- und/oder Nanomaterialien spezialisiert haben. Insbesondere kann es sich bei dem mindestens einen synthetischen oxidischen Material um ein nanoteiliges oxidisches Material handeln. Unter einem nanoteiligen Material ist dabei im Rahmen der vorliegenden Erfindung allgemein ein Material zu verstehen, dessen Partikel eine mittlere Partikelgröße unterhalb von 100 Nanometern aufweisen. Insbesondere kann es sich bei dem oxidischen Material um Siliciumoxid und/oder Aluminiumoxid und/oder Titanoxid handeln, beispielsweise Al₂O₃ und/oder TiO₂ und/oder SiO₂. Insbesondere können die genannten Oxide, welche auch als Mischoxide vorliegen können, in nanoteiliger Form vorliegen.

In einem weiteren Aspekt der vorliegenden Erfindung wird ein Verfahren zum Nachweis eines Analyten in einer Probe einer Körperflüssigkeit, insbesondere Vollblut, vorgeschlagen. Dabei wird ein diagnostisches Testelement in einer oder mehreren der oben beschriebenen Ausgestaltungen und/oder in einer oder mehreren der unten noch näher beschriebenen Ausführungsformen eingesetzt. Die Probe weist dabei ein Volumen von weniger als 2 Mikrolitern auf, insbesondere von weniger als 0,5 Mikrolitern und besonders bevorzugt von weniger als 0,3 Mikrolitern, beispielsweise von 100 Nanolitern. Insbesondere kann es sich bei der nachweisbaren Veränderung des mindestens einen Nachweisreagens des Testfeldes des verwendeten diagnostischen Testelements, wie oben ausgeführt, um eine optisch nachweisbare Veränderung handeln. In diesem Fall ist es besonders bevorzugt, wenn zum Nachweis der nachweisbaren Veränderung ein ortsauflösender optischer Detektor verwendet wird. Unter einem ortsauflösenden optischen Detektor ist dabei ein optischer Detektor zu verstehen, welcher eine Mehrzahl von optischen Sensoren aufweist, die nicht vollständig deckungsgleiche Bereiche der Nachweisseite der Nachweisschicht erfassen können. Insbesondere kann der ortsauflösende optische Detektor mindestens einen Bildsensor umfassen, also ein Array optischer Detektoren, welcher eindimensional oder auch zweidimensional ausgestaltet sein kann. Insbesondere kann der optische Detektor somit einen CCD- und/oder CMOS-Chip umfassen. Weiterhin kann der ortsauflösende optische Detektor mindestens ein optisches Element zur Abbildung der Nachweisseite und/oder der Nachweisschicht auf eine bildsensitive Fläche des ortsauflösenden optischen Detektors umfassen. Bei einem ortsauflösenden optischen Detektor und den genannten kleinen Probenvolumina machen sich die Vorteile der vorliegenden Erfindung, welche im Folgenden noch näher beschrieben werden, besonders bemerkbar, da bei herkömmlichen Nachweisschichten aufgrund der nachteiligen Benetzungseffekte und der Grobkörnigkeit der nachweisbaren Veränderung, insbesondere der optisch nachweisbaren Veränderung, hohe Unsicherheiten des Nachweises auftreten.

Das vorgeschlagene diagnostische Testelement, das vorgeschlagene Herstellungsverfahren und das vorgeschlagene Nachweisverfahren weisen gegenüber bekannten Vorrichtungen und Verfahren der genannten Art zahlreiche Vorteile auf. So besteht eine wichtige Grundlage der vorliegenden Erfindung in der Erkenntnis, dass bei der Verwendung kleiner Teilchen in Form von Partikeln zur Herstellung einer Nachweisschicht in der Regel Aggregatbildungen auftreten, sodass die Nachweisschicht nicht mehr von der geringen Teilchengröße der Primärpartikel profitieren kann. Ein Teststreifen, der gemäß bekannten Verfahren des Standes der Technik hergestellt wird, aus Stoffen mit den beschriebenen Teilchengrößen, wird folglich in der Regel entsprechend nur Teilchen in aggregiertem Zustand aufweisen. Die aus üblichen Herstellungsverfahren bekannten Teilchengrößen, welche in Pulvern als Ausgangsmaterial eingesetzt werden, sind somit nur Nennwerte, welche sich in der tatsächlichen Partikelgrößenverteilung in der Nachweisschicht in der Regel nicht wiederfinden. In der Regel muss bei der Herstellung der Nachweisschicht das Ausgangsmaterial (welches hier allgemein als Füllstoff bezeichnet wird oder welches mindestens einen Füllstoff enthalten kann) fein dispergiert werden. Bei einigen Füllstoff, wie beispielsweise Aerosilen und/oder Aeroxiden, muss dies in der Regel nicht besonders berücksichtigt werden, da derartige Stoffe vom Hersteller in vielen Fällen für eine leichte Diskbedienbarkeit optimiert wurden.

Erfindungsgemäß kann hingegen eine Herstellung des diagnostischen Testelements mit einer Nachweisschicht erfolgen, welche zu einer erheblich günstigeren Partikelgrößenverteilung in der fertigen Nachweisschicht führt. So können beispielsweise Ausgangspulver verwendet werden mit mittleren Partikelgrößen von beispielsweise bis zu 50 Nanometern, vorzugsweise bis zu 30 Nanometern, bei denen eine Dispergierung der Partikel vor der Herstellung der Nachweisschicht, beispielsweise vor dem Auftrag einer Dispersion zur Herstellung der Nachweisschicht auf das Trägerelement vorgesehen sein kann, sodass die Partikelgrößen die genannte Bedingung erfüllen. Dabei können beispielsweise die Partikelgrößen der Primärpartikel des Ausgangspulvers im Wesentlichen erhalten bleiben oder es kann lediglich in geringem Maße eine Agglomeration und/oder Aggregation der Primärpartikel während der Herstellung auftreten.

Dabei können als Ausgangsmaterialien zur Herstellung der Dispersion kommerziell erhältliche Materialien verwendet werden, beispielsweise kommerziell erhältliche Pulver, welche bereits die gewünschte Teilchengröße oder Partikelgrößenverteilung aufweisen. Alternativ oder zusätzlich ist es jedoch auch denkbar, dass zumindest Teile der Ausgangsstoffe, beispielsweise des mindestens einen Pulvers, vor der Erzeugung der Nachweisschicht, wie oben beschrieben, zunächst gemahlen werden, um eine entsprechende Partikelgrößenverteilung mit bevorzugten Korngrößen zu erzielen.

Darüber hinaus hat es sich gezeigt, dass, wie unten noch näher ausgeführt wird, nicht alle Materialien für ein derartiges Verfahren geeignet sind, da, in Abhängigkeit des ausgewählten Materials während der Dispergierung, derartige Materialien auch als Verdickungsmittel wirken können, so dass eine Gelbildung auftreten kann. Insbesondere können bestimmte Materialien ab beispielsweise einer Konzentration von mehr als 3 Gew.-%, insbesondere mehr als 5 Gew.-% oder sogar mehr als 20 Gew.-%, bezogen auf die Dispersion, unter Umständen als Verdickungsmittel wirken. Die oben genannten Materialien haben sich jedoch als besonders vorteilhaft erwiesen, da, in zumindest bei Konzentrationen von bis zu 3 Gew.-% in der Dispersion, vorzugsweise von mehr, beispielsweise von bis zu 5 Gew.-% oder bis zu 20 Gew.-%, eine derartige Verdickungswirkung nicht oder zumindest in der Regel nicht auftritt.

Die vorliegende Erfindung beruht weiterhin auf der Überwindung technischer Vorurteile, welche feinkörnigen Nachweisschichten häufig entgegengebracht werden. So war beispielsweise die Auswirkung kleiner Partikel auf eine Farbtiefe und eine Reaktionszeit in Nachweisschichten bislang unbekannt. Zur Erzielung einer erforderlichen Präzision müssen beispielsweise bei einem optischen Nachweis in der Regel Remissionsdifferenzen, auch bezeichnet als Reaktionshub, von mehr als 40 %, bevorzugt von mehr als 50% oder sogar mehr als 60 % relative Remission bezogen auf den Leerwert der trockenen Nachweisschicht, zwischen den Glukosekonzentrationen von 10 mg/dl und 600 mg/dl, welche typischerweise einen Messbereich bilden, erzielt werden. Unter einer Remission ist dabei allgemein die diffuse, ungerichtete Reflektion von Wellen zu verstehen, insbesondere von Licht, im Gegensatz zu einer regulären, gerichteten Reflektion. Die Remission wird dabei häufig auf die Oberfläche der Nachweisseite bezogen und auch als Remissionsgrad bezeichnet. Unter einem Remissionsgrad ist das Verhältnis der von einer Oberfläche remittierten Leuchtdichte zu einer Leuchtdichte einer Oberfläche in einem Referenzweiß zu verstehen. Die Remission ist bei optischen Testelementen, wie beispielsweise den im Stand der Technik beschriebenen Testelementen, eine übliche Messgröße und dem Fachmann auf diesem Gebiet bekannt.

Weiterhin müssen bei üblichen diagnostischen Testelementen in der Regel Reaktionszeiten von weniger als 10 Sekunden realisiert werden. Unter einer Reaktionszeit ist dabei eine Zeit zu verstehen, innerhalb derer sich ein im Wesentlichen stationärer Zustand nach Aufbringen der Probe auf das Testfeld eingestellt hat. Besonders bei den Reaktionszeiten bestand jedoch bislang die Befürchtung, dass dichter gepackte Inhaltsstoffe der Nachweisschicht eine längere Zeit zum Durchdringen und Auflösen durch die Probenflüssigkeit benötigen. Bei der Probenflüssigkeit kann es sich beispielsweise um Blut bzw. daraus nach Abtrennen der Erythrozyten gewonnenes Blutplasma handeln.

Um so überraschender war es, dass, wie unten noch näher ausgeführt wird, bei den bevorzugten Partikelgrößenverteilungen sich im Vergleich zu grobkörnigen Nachweisschichten der Remissionshub praktisch nicht änderte und auch die Reaktionszeit zumindest näherungsweise gleich blieb. Gleichzeitig ließen sich jedoch erheblich homogenere Benetzungen der Testfelder erzielen, wodurch sich, wie unten ebenfalls noch näher ausgeführt wird, insbesondere der Variations-Koeffizient deutlich verringern ließ. Unter Überwindung der genannten Vorurteile lassen sich somit diagnostische Testelemente erzeugen, welche eine erheblich höhere Präzision als herkömmliche diagnostische Testelemente aufweisen und welche gleichzeitig auch für die Vermessung sehr kleinvolumiger Proben geeignet sind, insbesondere unter Verwendung ortsauflösender optischer Detektoren.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Die Ausführungsbeispiele sind dabei zumindest teilweise in den Figuren schematisch dargestellt. Gleiche Bezugszeichen in den einzelnen Figuren bezeichnen dabei gleiche oder funktionsgleiche bzw. hinsichtlich ihrer Funktionen einander entsprechende Elemente. Die Erfindung ist nicht auf die Ausführungsbeispiele beschränkt.

Im Einzelnen zeigen:
- Figur 1: eine schematische Querschnittsdarstellung eines diagnostischen Testelements gemäß der vorliegenden Erfindung;
- Figuren 2A und 2B: Beispiele der Benetzung einer Testfeldoberfläche eines Testfelds eines herkömmlichen diagnostischen Testelements (Figur 2A) und eines erfindungsgemäßen diagnostischen Testelements (Figur 2B);
- Figur 3: Remissionskurven von diagnostischen Testelementen gemäß den Figuren 2A und 2B;
- Figur 4: Remissionskurven weiterer Ausführungsbeispiele diagnostischer Testelemente gemäß der vorliegenden Erfindung;
- Figur 5: Remissionskurven von Proben mit unterschiedlich dispergierten Inhaltsstoffen;
- Figuren 6A bis 6D: Mikroskopaufnahmen von Proben mit verschiedener Körnigkeit;
- Figuren 7A und 7B: Standardabweichungen der Grauwerte in den Figuren 6A bis 6D; und
- Figuren 8A und 8B: Autokorrelationsfunktionen der Grauwerte in den Figuren 6A bis 6D.

### Ausführungsbeispiele

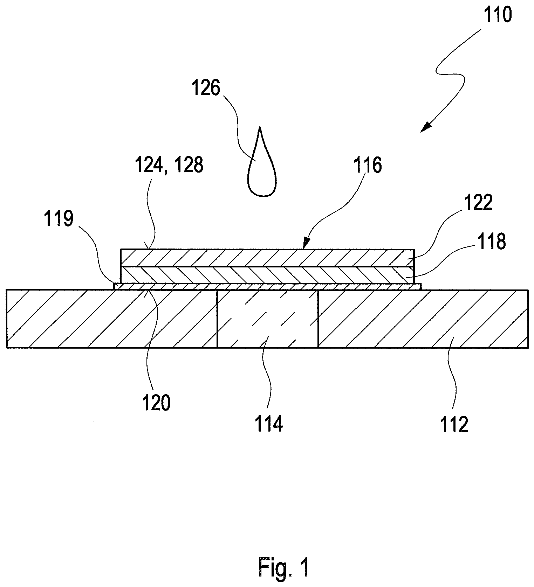

In Figur 1 ist schematisch ein möglicher Aufbau eines diagnostischen Testelements 110 in einer Schnittdarstellung gezeigt, welcher auch im Rahmen der vorliegenden Erfindung zum Einsatz kommen kann. Das diagnostische Testelement 110 umfasst in diesem Ausführungsbeispiel ein Trägerelement 112, welches beispielsweise als Streifen ausgestaltet sein kann. Insgesamt kann das diagnostische Testelement 110 somit als Teststreifen ausgestaltet werden.

Das Trägerelement 112 umfasst mindestens einen transparenten Bereich 114. Im Bereich des transparenten Bereichs 114 ist auf das Trägerelement 112 ein Schichtaufbau aufgebracht, der den transparenten Bereich 114 ganz oder teilweise bedecken kann. Dieser umfasst in dem dargestellten Ausführungsbeispiel zwei Schichten und bildet ein Testfeld 116. Dieses Testfeld 116 umfasst in dem dargestellten Ausführungsbeispiel exemplarisch eine Nachweisschicht 118 mit einer dem Trägerelement 112 und dem transparenten Bereich 114 zuweisenden Nachweisseite 120. Weiterhin umfasst das Testfeld 116 in dem dargestellten Ausführungsbeispiel eine Abtrennschicht 122 auf der dem Trägerelement 112 abgewandten Seite der Nachweisschicht 118. Diese Abtrennschicht 122 dient zum Abtrennen grober Bestandteile einer Probe 126 einer Körperflüssigkeit, welche auf einer Aufgabenseite 128 auf eine Testfeldoberfläche 124 aufgebracht werden kann.

Der transparente Bereich 114 kann beispielsweise einfach als Öffnung, beispielsweise als Loch, in dem Trägerelement 112 ausgestaltet sein. Insbesondere in diesem Fall, jedoch auch in anderen Ausführungsformen, kann zusätzlich auf das Trägerelement 112 eine Trägerfolie oder eine andere Art von Träger aufgebracht werden, vorzugsweise eine transparente Trägerfolie. Diese optionale Trägerfolie ist in Figur 1 mit der Bezugsziffer 119 bezeichnet. Diese Trägerfolie 119 kann beispielsweise zwischen das Trägerelement 112 und die Nachweisschicht 118 in dem in Figur 1 gezeigten Schichtaufbau eingebracht sein. Beispielsweise kann die Trägerfolie 119 als Teil eines Reaktionsfilms ausgestaltet sein, wobei auf die Trägerfolie 119 die mindestens eine Nachweisschicht 118 und optional die mindestens eine Abtrennschicht 122 aufgebracht werden, beispielsweise mittels eines Druckprozesses und/oder eines Rakelverfahrens. Anschließend wird dieser Reaktionsfilm dann aufdas eigentliche Trägerelement 112 mit dem transparenten Bereich 114 aufgebracht, so dass die Nachweisschicht 118 durch den transparenten Bereich 114 hindurch erkennbar ist. Alternativ kann der transparente Bereich 114 jedoch auch ganz oder teilweise mit einem transparenten Material ausgefüllt sein, beispielsweise einem transparenten Kunststoffmaterial, und/oder das gesamte Trägerelement 112 kann als transparentes Trägerelement ausgestaltet sein. Insbesondere in diesem Fall, jedoch auch in anderen Fällen, kann der Schichtaufbau mit der mindestens einen Nachweisschicht 118 und optional der mindestens einen Abtrennschicht 122 auch direkt auf das Trägerelement 112 aufgebracht werden. Alternativ kann auch hier wieder einen Reaktionsfilm gemäß den obigen Ausführungen eingesetzt werden, welcher auf das Trägerelement 112 aufgebracht wird.

Es wird darauf hingewiesen, dass der in Figur 1 dargestellte Aufbau des diagnostischen Testelements lediglich exemplarisch zu verstehen ist und dass auch andere Arten von Aufbauten möglich sind. So können beispielsweise mehrere Nachweisschichten 118 und/oder mehrere Abtrennschichten 122 vorgesehen sein. Weiterhin kann der in Figur 1 gezeigte Aufbau durch verschiedene weitere Elemente, die nicht dargestellt sind, ergänzt werden. So kann beispielsweise auf der Testfeldoberfläche 124 ein Spreitnetz vorgesehen sein. Weiterhin können Teile der Testfeldoberfläche 124 abgedeckt sein, beispielsweise mit einem hydrophoben Material, beispielsweise um lediglich einen Teil der Aufgabenseite 128 für eine Beaufschlagung mit der Probe 126 zugänglich zu gestalten. Für mögliche Ausgestaltungen des diagnostischen Testelements 110 kann beispielsweise auf die oben genannte EP 0 821 234 B1 oder auf andere bekannten Testreifenaufbauten verwiesen werden.

### Beispiel 1

Die vorliegende Erfindung bezieht sich im Wesentlichen auf die Ausgestaltung und Herstellung der Nachweisschicht 118. Um diagnostische Testelemente 110 mit der oben beschriebenen erfindungsgemäßen Partikelgrößenverteilung mit herkömmlichen diagnostischen Testelementen zu vergleichen, könnten grundsätzlich Schichtaufbauten verwendet werden, wie sie beispielsweise in EP 0 821 234 B1 beschrieben sind. Im vorliegenden Beispiel werden jedoch werden Schichtaufbauten des Testfelds 116 verwendet, die wie folgt hergestellt sind:

### Probe A:

Als Vergleichsprobe (Probe A) wird ein diagnostisches Testelement 110 entsprechend dem folgenden Aufbau hergestellt:

### a) Nachweisschicht:

Zur Herstellung einer Dispersion für die Nachweisschicht 118 werden zunächst zwei Teillösungen (Teillösung 1 und 2) hergestellt, diese dann zu einem Teilansatz vereinigt. Der Begriff "Lösung" wird in diesem Zusammenhang unabhängig davon verwendet, ob tatsächlich eine echte Lösung vorliegt oder lediglich beispielsweise eine Dispersion. Eine Enzymlösung wird hergestellt, und der Teilansatz 1 und die Enzymlösung werden gemischt, so dass eine Beschichtungsmasse entsteht. Hierzu wird wie folgt vorgegangen:
Teillösung 1: 0,34 g Xanthangum werden in 35,5 g 0,02 M Glycerin-3-phosphat-Puffer von pH 6,5 24 h vorgequollen und mit 5,0 g Polyvinylpropionat-Dispersion gemischt.
Teillösung 2: 5,2 g Transpafill werden in 21,5 g Wasser 10 min mit einem Ultraturrax dispergiert.

Teilansatz 1: Beide Teillösungen werden vereinigt und nach Zugabe von 0,15 g Tetraethylammoniumchlorid, 0,17 g N-Octanoyl-N-methyl-glucamid, 0,06 g N-Methyl-N-octadecenyl-taurat ("Geropon T 77") und 0,88 g PVP (MG 25.000) 1 h mit einem Flügelrührer mäßig gerührt. Dann werden der Reihe nach folgende Teillösungen hinzugegeben:
- 0,10 g Bis-(2-hydroxyethyl)-(4-hydrox-iminocyclohexa-2,5-dienylidin)-ammoniumchlorid in 1,5 g Wasser,
- 0,65 g 2,18-Phosphormolybdänsäure-hexanatriumsalz in 1,5 g Wasser, worauf der pH mit NaOH auf 6,7 eingestellt wird.

Enzymlösung: Zu 25,6 g 0,1 M Glycerin-3-phosphat-Puffer von pH 6,5 werden 5 mg PQQ-di-Natriumsalz und 0,28 g GDH (Mutante 31) sowie 0,16 g einer 1 M CaCl₂-Lösung gegeben und > 3 h gerührt.

Teilansatz 1 und Enzymlösung werden gemischt, mit einer Lösung von 20 mg K₃[Fe(CN)₆] in 0,4 g Wasser sowie 1,0 g 2-Methyl-2-butanol versetzt und 30 min gerührt. Hierbei entsteht eine Beschichtungsmasse zur Herstellung der Nachweisschicht 118.

Die derart hergestellte Beschichtungsmasse wird mit einem Flächengewicht von 90 g/m² auf eine Trägerfolie 119 in Form einer Polycarbonatfolie mit einer Dicke von 125 Mikrometern aufgetragen und getrocknet.

Bei Transpafill® handelt es sich um ein kommerziell erhältliches Natriumaluminiumsilikatpulver der Evonik Industries AG. Die präzisionsverbessernde Wirkung von N-Methyl-N-octadecenyl-taurat ("Geropon T 77") wurde in EP 0 995 994 beschrieben.

### b) Abtrennschicht:

Auch zur Herstellung der Abtrennschicht 122 werden in dem vorliegenden Ausführungsbeispiel zunächst zwei Teillösungen (Teillösung 1 und Teillösung 2) hergestellt und diese dann vereinigt. Hierbei wird wie folgt vorgegangen:
Teillösung 1: Eine Aufschlämmung von 1,37 g Gantrez S 97 in 13,5 g Wasser wird mit 2,2 g 16 %-iger NaOH versetzt und über Nacht vorgequollen. Dann werden 0,40 g Tetraethylammoniumchlorid, 0,34 g N-Octanoyl-N-methyl-glucamid, 0,06 g N-Methyl-N-octadecenyl-taurat ("Geropon T 77") und 1,87 g PVP (MG 25.000) hinzugegeben und 1 h gerührt.
Teil"lösung" 2: 14,3 g Titandioxid E 1171 der Fa. Kronos und 1,95 g Fällungskieselsäure FK 320 der Fa. Degussa werden in 36,4 g Wasser 10 min mit einem Ultraturrax dispergiert.

Nach Vereinigen der Teillösungen werden 5,7 g Polyvinylpropionat-Dispersion, 0,15 g Bis-(2-hydroxyethyl)-(4-hydrox-iminocyclohexa-2,5-dienylidin)-ammoniumchlorid in 4,2 g Wasser, 1,85 g 2,18-Phosphormolybdänsäure-hexanatriumsalz in 4,2 g Wasser, 10 mg K₃[Fe(CN)₆] in 0,4 g Wasser hinzugefügt und mit NaOH auf pH 6,8 gestellt. Nach Zugabe von 1,0 g 2-Methyl-2-butanol noch 1 h gerührt.

Bei der Bezeichnung Gantrez® handelt es sich um eine Produktbezeichnung der ISP International Speciality Products, Köln, Deutschland. Chemisch handelt es sich dabei um ein Copolymer von Maleinsäure und Methylvinylether.

Die derart durch Vereinigung der Teillösungen 1 und 2 hergestellte Beschichtungsmasse wird dann mit einem Flächengewicht von 45 g/m² auf die wie vorstehend beschriebene erst-beschichtete Trägerfolie 119 aus Polycarbonat, also auf die Nachweisschicht 118, aufgetragen und getrocknet.

### Probe B:

Zur Herstellung eines erfindungsgemäßen diagnostischen Testelements 110 wird in der Nachweisschicht 118, der im Wesentlichen für die Grobkörnigkeit dieser Nachweisschicht 118 verantwortliche Rohstoff Transpafill® einem Mahlprozess unterworfen. Optional kann auch der ebenfalls grobkörnige Rohstoff Kieselgur in der Abtrennschicht 122, welcher als Fällungskieselsäure wirkt, einem Mahlprozess unterworfen werden. Nicht gemahlen werden sollte hingegen in der Abtrennschicht 122 das Titandioxid, welches als weißes Pigment dient und somit eine Reflektivität für das eingestrahlte Licht, beispielsweise Licht der Wellenlänge von 660 Nanometern, aufweisen sollte. Dieses Licht wird beispielsweise durch den transparenten Bereich 114 hindurch eingestrahlt, durchstrahlt die Nachweisschicht 118 und wird an der Abtrennschicht 122 reflektiert, so dass die Abtrennschicht 122 in dem in Figur 1 dargestellten Ausführungsbeispiel gleichzeitig als Reflexionsschicht dienen kann.

Um die oben genannten grobkörnigen Füllstoffe Transpafill® und optional Fällungskieselsäure zu zerkleinern, werden diese einem Nassmahlschritt unterzogen. Dies kann mit einer Rührwerkskugelmühle erfolgen, beispielsweise für 20 Minuten, was entsprechend einer Messung zu einer Partikelgröße d₅₀ von ca. 0,3 Mikrometern und einer Partikelgröße d₉₀ von ca. 0,5 Mikrometern führt. Mit dem Wert d₉₀ wird die Partikelgröße bezeichnet, für die 90% der Partikel feiner als der Wert d₉₀ sind.

Mit den derart hergestellten Proben A und Proben B lassen sich verschiedene Vergleichsversuche durchführen. Mittels dieser Vergleichsversuche kann insbesondere das Vorurteil ausgeräumt werden, dass bei dichter gepackten Inhaltsstoffen eine längere Zeit zum Durchdringen und damit ein Auflösen durch die Probenflüssigkeit benötigt werden.

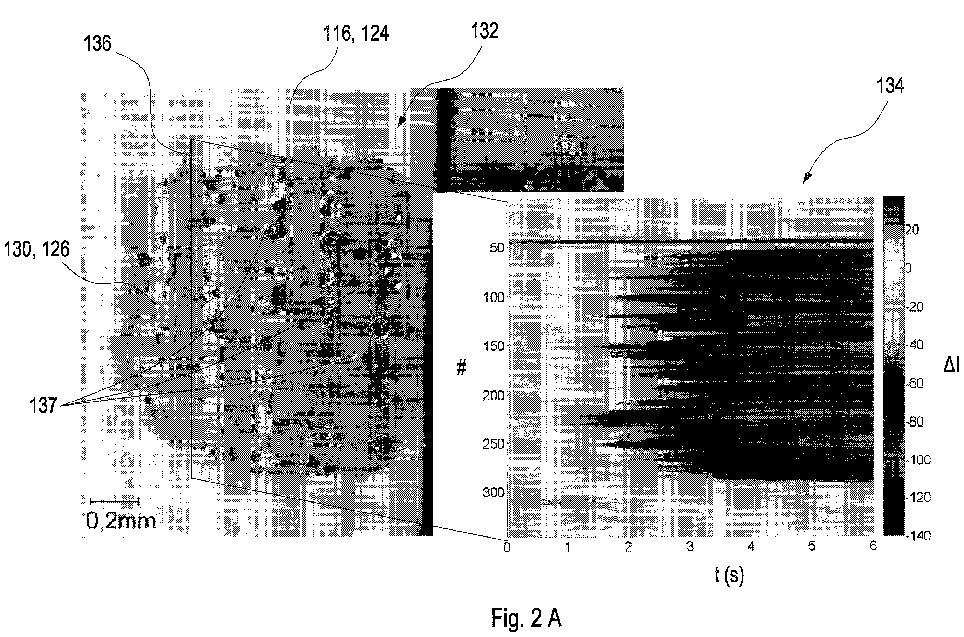

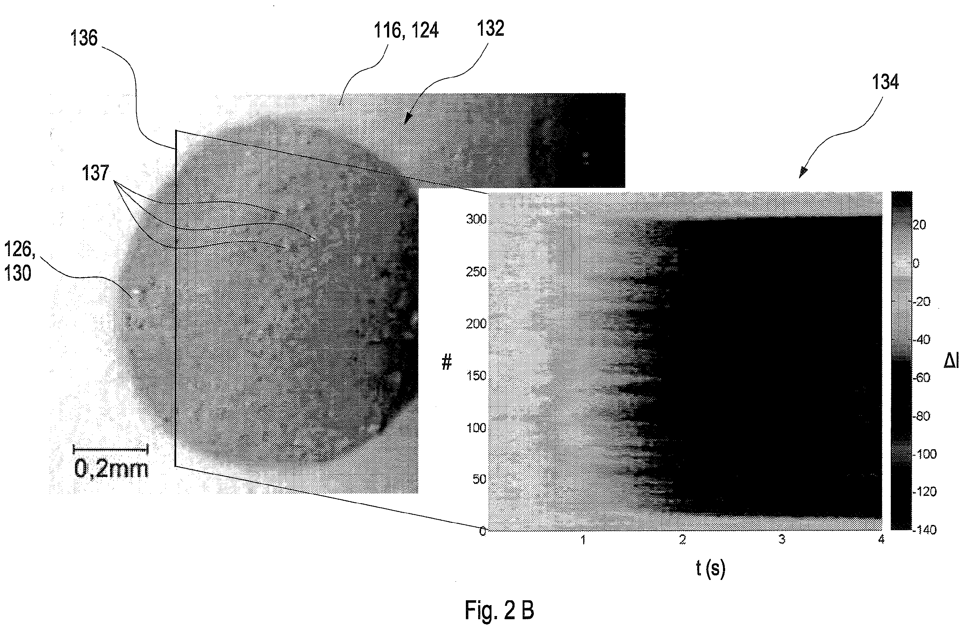

In den Figuren 2A und 2B sind Benetzungsversuche dargestellt, welche an Proben vom Typ A (Figur 2A) und Proben vom Typ B (Figur 2B) durchgeführt wurden. Diese Versuche zeigen zunächst den Einfluss des Mahlschritts auf die Benetzung. Die Vergleichsversuche zeigen jeweils Testfelder 116 mit einer Testfeldoberfläche 124, auf welche ein Tropfen 130 der Probe 126 aufgebracht ist. Dabei zeigen die Teilbilder 132 in den Figuren 2A und 2B jeweils Mikroskopaufnahmen der Testfeldoberfläche 124, wohingegen die Teilbilder 134 eine Grauwertänderung in den Mikroskopaufnahmen 132 entlang einer Schnittlinie 136 durch den Tropfen 130 der Probe 126 zeigen. Als Probe 126 wurde dabei eine Testflüssigkeit mit einer Konzentration von 50 mg/dl Glucose verwendet.

In den Teilbildern 134 ist auf der vertikalen Achse in willkürlichen Einheiten die mit # bezeichnete Pixelposition entlang der Schnittlinie 136 aufgetragen. Die horizontale Achse gibt die Zeit t in Sekunden nach Aufbringen der Probe 126 an. In dem Teilbild 134 sind dabei jeweils die Grauwertänderungen dargestellt. Rechts in diesem Teilbild ist eine Skala dargestellt, welche die Grauwertänderungen ΔI in willkürlichen Einheiten angibt. Dabei zeigt Figur 2A eine Testfeldoberfläche 124 der Probe A, also eines Testfeldmaterials, wie es in handelsüblichen Teststreifen derzeit verwendet wird. Figur 2B zeigt hingeben ein Testfeld 116 mit dem Testfeldmaterial gemäß der Probe B gemäß der vorliegenden Erfindung.

Ohne auf numerische Details der Messung einzugehen, zeigen insbesondere die Teilbilder 134 der Grauwertänderung in den Figuren 2A und 2B in unmittelbarem Vergleich, dass das Mahlen des Testfeldmaterials zu einer deutlich homogeneren zeitlichen Änderung der Remissionscharakteristik entlang der Schnittlinie 136 führt. Dementsprechend erfolgt das Einsetzen der Reaktion, welche für den Nachweis des nachzuweisenden Analyten spezifisch ist, in dem Ausführungsbeispiel gemäß Figur 2B entlang der Schnittlinie 136 nahezu zeitgleich, wohingegen in dem Versuch mit ungemahlenem Testfeldmaterial gemäß Figur 2A ein starker zeitlicher Versatz des Reaktionseinsatzes zu verzeichnen ist. So kann ein zeitlicher Versatz zwischen einzelnen Orten entlang der Schnittlinie 136 eintreten, welcher bis zu 3 Sekunden oder mehr betragen kann. Auch sind Orte entlang der Schnittlinie 136 zu beobachten, an welchen die Reaktion instantan eintritt, sowie Orte, an welchen die Reaktion überhaupt nicht abzulaufen scheint.

Weiterhin ist in den Figuren 2A und 2B jeweils eine Mehrzahl von Partikeln 137 in der Nachweisschicht 118 erkennbar. Sichtbar in den Mikroskopaufnahmen in den Teilbildern 132 ist dabei jeweils nahezu ausschließlich die Nachweisschicht 118, da Lichtstrahlen, die durch den transparenten Bereich 114 in die Nachweisschicht 118 eintreten, spätestens an den Pigmenten der Abtrennschicht 122, insbesondere Titandioxid-Pigmenten, reflektiert werden.

Dabei ist deutlich erkennbar, dass die Partikel 137 in der herkömmlichen Probe A gemäß Figur 2A erheblich größer sind und eine breitere Partikelgrößenverteilung aufweisen als die Partikel 137 in der erfindungsgemäßen Probe B gemäß Figur 2B. Mittels einer derartigen Mikroskopaufnahme gemäß dem Teilbild 132 in den Figuren 2A und 2B lässt sich ohne weiteres auch durch eine Bilderkennung und eine automatische Erkennung von Partikeln bei einer entsprechenden Vergrößerung eine Partikelgrößenverteilung erstellen. Alternativ oder zusätzlich könnte auch eine Grauwertänderung gemäß den Teilbildern 134 herangezogen werden. Derartige Auswertungsverfahren mit einer automatischen Bilderkennung sind dem Fachmann aus dem Bereich der Bildverarbeitung grundsätzlich bekannt.

Insgesamt lässt sich somit als erster positiver Effekt der Verwendung eines gemahlenen Testfeldmaterials eine Vergleichmäßigung des Ablaufs der Analyt-spezifischen Reaktion beobachten. Weiterhin ist, wie aus den Figuren 2A und 2B deutlich hervorgeht, insgesamt eine Homogenisierung der Reaktion über die benetzte Testfeldoberfläche 124 hinweg zu verzeichnen.

Weiterhin bleibt insgesamt bei den Versuchen die Reaktionszeit bei gemahlenen und ungemahlenen Proben insgesamt im Mittel näherungsweise gleich. So wurde in allen Fällen eine Reaktionszeit von ca. 6 bis 7 Sekunden festgestellt. Wie aus den oben beschriebenen Ergebnissen hervorgeht, ist jedoch die ortsaufgelöste, lokale Reaktionszeit bei gemahlenen Testfeldmaterialien stark vergleichmäßigt, so dass die lokalen Schwankungen der Reaktionszeiten durch das gemahlene Testfeldmaterial erheblich verbessert werden können.

In einem weiteren Versuch werden mit den Proben A und B Versuche zum Remissionshub durchgeführt. Gemäß dem oben skizzierten Vorurteil, dass gemahlene Testfeldmaterialien zu einer unvollständigen Durchdringung der Nachweisschicht 118 führen, müsste bei Proben vom Typ B eine deutliche Verringerung des Remissionshubs festzustellen sein, da lediglich ein kleinerer Bereich der Nachweisschicht 118 von der Probe 126 durchdrungen werden sollte und somit für die Nachweisreaktion zur Verfügung steht.

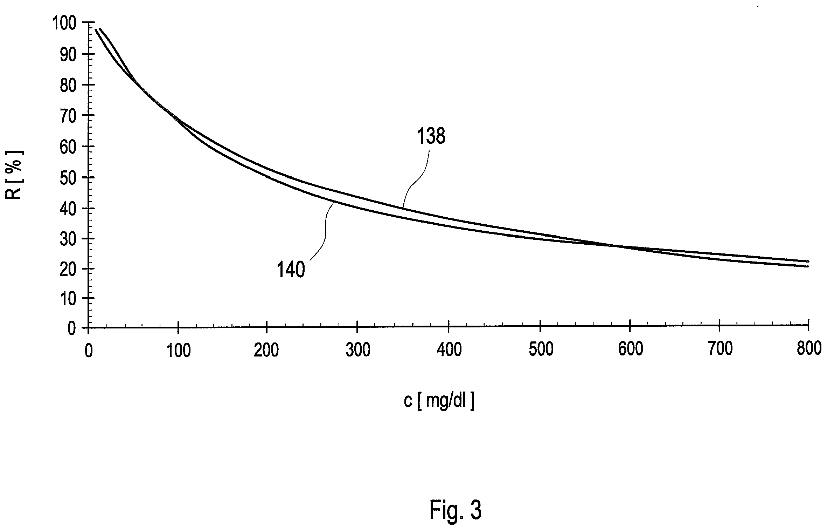

Die Ergebnisse dieser Remissionsmessungen sind in Figur 3 dargestellt. Auf der horizontalen Achse ist dabei die Konzentration c der Glucose in der Probe 126 dargestellt, wohingegen auf der vertikalen Achse die relative Remission R aufgetragen ist. Es wurde als Probe 126 EDTA-Venenblut verwendet, wobei die Konzentrationen von Glucose in dieser Testflüssigkeit variiert wurden. Die Kurve 138 in Figur 3 zeigt dabei die Remissionen, welche an einem herkömmlichen diagnostischen Testelement, also einer Probe vom Typ A, gemessen wurden, wohingegen die Kurve 140 Remissionen einer erfindungsgemäßen Probe vom Typ B zeigt. Wie sich aus diesen Darstellungen erkennen lässt, ändert sich der Remissionshub, also die Änderung der Remission über den gesamten Messbereich, welcher typischerweise zwischen 10 und 600 mg/dl liegt, praktisch nicht. Das Nassmahlen der Füllstoffe führt somit nicht zu einer Verschlechterung der optischen Eigenschaften und/oder der Nachweiseigenschaften.

Somit zeigen die in den Figuren 1 bis 3 dargestellten Versuche deutlich, dass mit der Verwendung gemahlener Füllstoffe keine Verschlechterung der Eigenschaften der diagnostischen Testelemente 110 in Form einer Verlängerung der Reaktionszeit oder in Form einer Verschlechterung des Remissionshubs einhergeht. Gleichzeitig lässt sich jedoch, wie die Figuren 2A und 2B deutlich belegen, die Homogenität und die Präzision der Messungen durch die Verwendung der gemahlenen Testfeldchemie deutlich verbessern. Bereits in einem Messgerät vom Typ Accu-Chek® Active wurde bei mehreren Messungen eine Verbesserung des Variationskoeffizienten VK (auch als "coefficient of variation" bezeichnet, CV), welcher das Verhältnis zwischen Standardabweichung und Mittelwert der Messungen angibt, von 1,5 auf 1,2 % beim Übergang von Proben vom Typ A zu Proben vom Typ B verzeichnet.

Alternativ zu dem oben anhand der Probe B beschriebenen Nassmahlen kann das Mahlen auch beispielsweise, alternativ oder zusätzlich, mit einem Trockenmahlschritt erfolgen. Dementsprechend kann beispielsweise eine Luftstrahlmühle verwendet werden, mittels derer grundsätzlich Teilchengrößen bis zu beispielsweise 100 Nanometern erreichbar sind.

Weiterhin wurden Versuche durchgeführt, bei denen die vollständigen Beschichtungsmassen für die Nachweisschicht 118 und die Abtrennschicht 122 gemahlen wurde. In der Nachweisschicht 118 wurde dabei das Nachweisreagenz, insbesondere das Enzym, wegen des Energieeintrags beim Mahlverfahren nicht mitgemahlen. Derartige Prozesse brachten jedoch insgesamt keine oder lediglich eine geringfügige Verbesserung der Homogenität, des Remissionshubs und der Reaktionszeit. Das Mahlen der kompletten Beschichtungsmassen birgt also keinen Vorteil gegenüber dem Mahlen der Füllstoff-Voransätze. Letzteres ist produktionstechnisch jedoch erheblich einfacher, da auf Vorrat gemahlen werden kann.

### Beispiel 2

Das Mahlen der Rohstoffe für die Nachweisschicht 118 stellt einen zusätzlichen Prozessschritt dar und kann die Kosten der diagnostischen Testelemente 110 in die Höhe treiben. Daher werden in einer zweiten Phase Rohstoffe untersucht, die kommerziell erhältlich sind und die von vorneherein eine mittlere Teilchengröße im Bereich << 1 Mikrometer mit sich bringen. Hier bietet sich unter anderem die Produktpalette der so genannten Aerosiltypen der Evonik Industries AG an. Hierbei handelt es sich um hydrophile, nanoteilige Oxide, insbesondere Metalloxide.

So wurden, als Ersatz des oben beschriebenen Transpafill® in der Probe B folgende Ersatzstoff identifiziert:

**Tabelle 1: Beispiele weiterer möglicher Ersatzstoffe für Transpafill®.**

| Material: | Typ: | Mittlere Partikelgröße: |
|---|---|---|
| SiO₂: | Beim Dispergieren kaum verdickend: Aerosil EG 50, Aerosil 90 | 20 nm |
| | Beim Dispergieren stark verdickend: Aerosil 200, Aerosil COK 84 | |
| TiO₂: | Aeroxide TiO₂ P 25 | 21 nm |
| Al₂O₃ : | Aeroxide Alu 65 | 17 nm |

Mit "beim Dispergieren verdickend" bzw. "beim Dispergieren nicht verdickend" ist dabei die Eigenschaft bestimmter Stoffe bezeichnet, welche experimentell feststellbar ist, dass diese Stoffe während des Dispergiervorgangs eine Verdickungswirkung der Dispersion herbeiführen.

Für den Einsatz dieser Ersatzstoffe besteht zunächst natürlich, aufgrund des oben genannten Vorurteils, die Befürchtung, dass die Poren nun endgültig eine zu geringe Größe aufweisen könnten, um eine Durchdringung der Nachweisschicht 118 zu ermöglichen, so dass der Remissionshub und die Reaktionszeiten im Vergleich zu Standard-Proben eine Verschlechterung erfahren.

Dementsprechend werden Proben hergestellt, bei welchen im Vergleich zur Probe A oben das Transpafill® in der Nachweisschicht 118 1 : 1 durch folgende Materialien ersetzt wurde:
Probe C:
   SiO₂, Aerosil COK 84 (Mischoxid mit 10 % Al₂O₃), mittlere Partikelgröße 20 nm
Probe D:
   TiO₂, Aeroxide TiO₂ P 25, mittlere Partikelgröße 21 Nanometer
   und
Probe E:
   Al₂O₃, Aeroxide Alu 65, mittlere Partikelgröße 17 Nanometer
Probe F:
   In einer vierten Probe in diesem zweiten Beispiel wird im Vergleich zur Probe A anstelle des Transpafill® eine nassgemahlene Mischung von Fällungskieselsäure und Titandioxid mit einer insgesamt mittleren Partikelgröße von 0,3 Mikrometern eingesetzt.

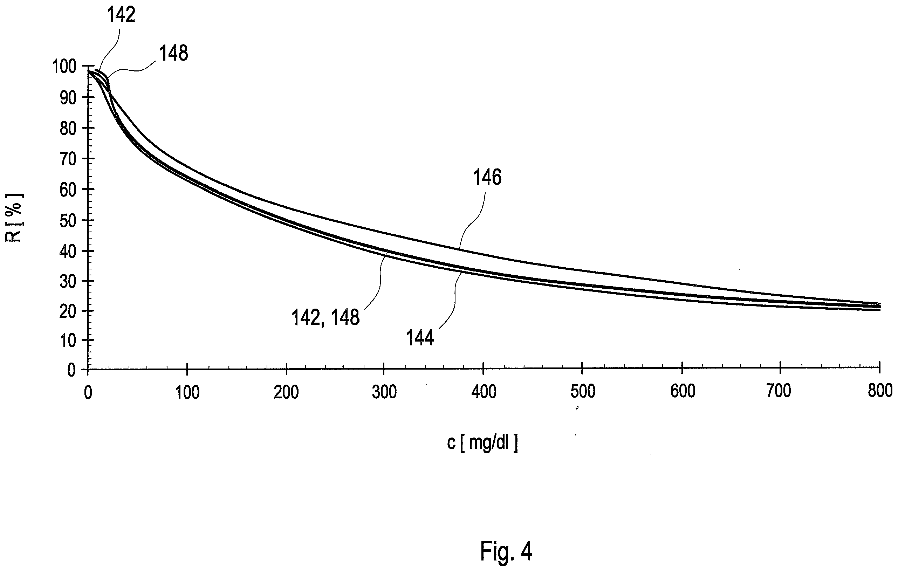

An diesen Proben werden teilweise wiederum Remissionsmessungen durchgeführt, analog zum Experiment gemäß Figur 3. Die Ergebnisse dieser Messungen sind in Figur 4, in analoger Darstellung zur Figur 3, aufgetragen. Dabei bezeichnet die Kurve 142 die Remission der Probe A, die Kurve 144 die Remission der Probe D, die Kurve 146 die Remission der Probe E, und die Kurve 148 die Remission der Probe F.

Zunächst wurde festgestellt, dass die Reaktionszeiten bei allen Proben um 6 Sekunden lagen und somit unverändert gegenüber der Vergleichsprobe A. Weiterhin bleiben auch die Remissionshübe, wie in Figur 4 deutlich erkennbar, über den Messbereich hinweg im Wesentlichen gleich. Die Kurven 142 und 148 sind dabei in weiten Teilen sogar in Figur 4 überlappend.

Allerdings zeigt sich bei diesem Versuchen, dass die feinstteiligen Füllstoffe nahezu auf ihre Primärteilchengröße dispergiert werden sollten, um eine Agglomeration und/oder eine Aggregatbildung zu vermeiden. Zu diesem Zweck werden herkömmliche Dissolver eingesetzt, wobei beispielsweise auf Geräte der Firmen Kinematica AG vom Typ Polytron® oder Megatron® zurückgegriffen werden kann, oder auf Geräte der Firma IKA Maschinenbau, beispielsweise vom Typ Ultra-Turrax®.

Bei SiO₂-Aerosil-Typen, wie beispielsweise den Proben C gemäß der obigen Beschreibung, zeigt sich, dass Dissolver nur schwer eingesetzt werden können. Dissolver werden somit vorzugsweise für Proben vom Typ D und E eingesetzt, also für Titanoxide und Aluminiumoxide, insbesondere mit nominellen mittleren Partikelgrößen der Ausgangspulver von 30 Nanometern oder weniger. Bei Proben vom Typ C führt hingegen die Verwendung von Dissolvern aufgrund der Verdickungswirkung der SiO₂-Aerosil-Typen und einer Gelbildung nur bis zu Konzentrationen von ca. 3 Gew.-% in der nassen Ausgangsmischung zu einer Dispergierung. Hier lassen sich jedoch Rührer mit niedrigerer Scherrate einsetzen, welche aber in Experimenten nahezu gleiche Ergebnisse wie die Kurven in Figur 4 lieferten. Dies weist darauf hin, dass die Aerosile auf Dispergierbarkeit optimiert wurden. Bei Aeroxide TiO₂ P 25 und Aeroxide Alu 65 trat bei Experimenten die Verdickung lediglich unwesentlich ein.

Alternativ oder zusätzlich zu den oben genannten Füllstoffen, gemahlen oder kommerziell bereits als Nanoteilchen verfügbar, wurde nach weiteren Substanzen gesucht, die als Füllstoffe in der Nachweisschicht 118 einsetzbar sind, beispielsweise als Ersatz des Transpafill® in der oben beschriebenen Probe A. Dabei wurde neben den oben beschriebenen Aerosil-Typen unter anderem folgende Substanzen ermittelt:
- Kaolin
- Pulverglas (Fa. TROVOtech, Wolfen)
- Fällungskieselsäure
- Calciumsulfat x 2 H₂O
- Natriumaluminiumsilikate wie etwa Sipernat 44 MS (Degussa/Evonik).

Diese Füllstoffe sind entweder bereits in der gewünschten Partikelgröße oder Partikelgrößenverteilung verfügbar oder können durch Mahlen auf die erforderliche Teilchengröße bzw. Partikelgrößenverteilung prozessiert werden.

Die obigen Experimente zeigen im Wesentlichen, dass herstellerseitig die Aerosil/Aeroxide-Typen auf leichte Dispergierbarkeit getrimmt wurden, so dass die Einarbeitung weitgehend scherkraft-unabhängig ist.

Um die Notwendigkeit einer Dispergierung bis auf Primärteilchengröße genauer zu untersuchen, werden weitere Versuche vorgenommen. Hierfür wird die obige Probe D nochmals auf verschiedene Weise untersucht. Dafür werden Aeroxide TiO₂ P 25 einmal mit einem Dissolver (Probe G) und einmal mit einem Propellerrührer mit niedrigen Drehzahlen dispergiert. Die Zugabe von Aeroxide TiO₂ P 25 erfolgt einmal nach vorherigem Anteigen mit Wasser zu einer Paste (Probe H) und einmal durch festes Eintragen in die Verdickerlösung (Xanthangum) (Probe I). Als Vergleichsprobe wird nach wie vor die Probe A gemäß der obigen Beschreibung verwendet. Insgesamt ergeben sich damit für den im Folgenden beschriebenen Versuch folgende Proben:
- Probe A':: wie Probe A oben
- Probe G:: wie Probe D, jedoch Dispergierung von Aeroxide TiO₂ P 25 mit Dissolver,
- Probe H:: wie Probe D, jedoch Dispergierung Aeroxide TiO₂ P 25 mit Propellerrührer mit niedrigen Drehzahlen, Zugabe nach vorherigem Anteigen mit Wasser zu einer Paste, und
- Probe I:: wie Probe D, jedoch Dispergierung Aeroxide TiO₂ P 25 mit Propellerrührer mit niedrigen Drehzahlen durch festes Eintragen in die Verdickerlösung (Xanthangum).

Auf diese Weise werden Proben G bis I hergestellt, in welchen Transpafill® gewichtsmäßig 1 : 1 durch Aeroxide TiO₂ P 25 ersetzt ist. Ansonsten erfolgt die Herstellung der diagnostischen Testelemente 110 wie oben beschrieben.

Mit diesen Proben von diagnostischen Testelementen 110 in Form von Teststreifen werden am kommerziellen Blutzuckermesssystem Accu-Chek Active mit 15 verschiedenen Glucosekonzentrationen in EDTA-Venenblut Messungen durchgeführt, wobei n = 10 Einzelmessungen pro Konzentration ausgewertet werden.

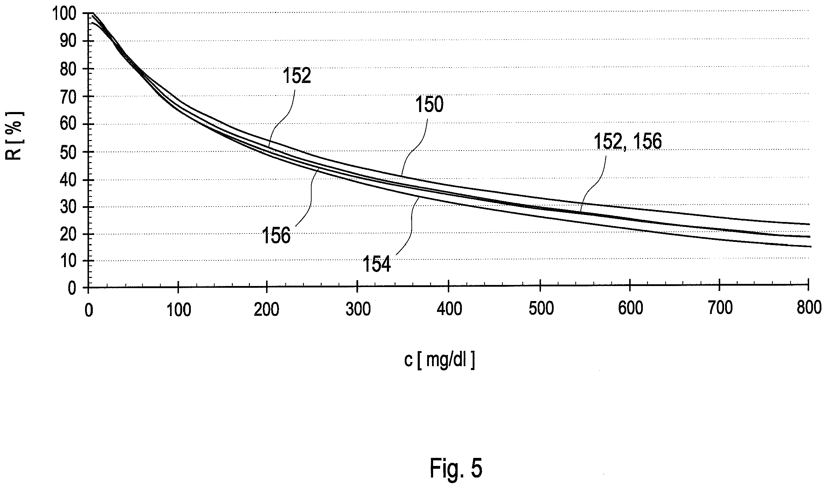

In Figur 5 sind, in einer zu Figur 4 analogen Darstellung, Remissionskurven zu diesen Proben A', G, H und I dargestellt. Dabei bezeichnet die Kurve 150 Remissionsmessungen der Probe A', die Kurve 152 Remissionsmessungen der Probe G, die Kurve 154 Remissionsmessungen der Probe H und die Kurve 156 Remissionsmessungen zur Probe I.

Die Messkurven zeigen, dass die Farbtiefe bei den vier verschiedenen Beschichtungen nahezu gleich ist. Auch die Reaktionsgeschwindigkeit liegt bei allen Proben im Bereich von 6 bis 8 Sekunden.

Dies zeigt, dass das Aeroxide TiO₂ P 25 in allen drei Fällen, also in den Proben G, H und I, feindispers vorliegt, da Aggregate des TiO₂ Pigmentcharakter haben und die Reaktionsfarbe abschwächen würden. Eine Farbabschwächung würde dadurch bedingt, dass im Fall einer Aggregatbildung ein Pigment in der Nachweisschicht 118 vorliegen würde. Feindispergiertes TiO₂ ist hingegen kein Pigment, da in diesem Fall die Teilchengröße kleiner als die Lichtwellenlänge ist. Diese Eigenschaft wird beispielsweise in Sonnenschutzmitteln ausgenutzt.

Der wesentliche Vorteil der Nachweisfilme mit feinkörnigen Füllstoffen besteht in der deutlich verbesserten Homogenität der Reaktionsfarben, was daher die Vermessung kleinerer Flächen und damit kleinerer Blutvolumina erlaubt.

Dies lässt sich nochmals in einem Vergleichsexperiment zeigen, in welchem verschiedene Proben untersucht werden. Hierfür werden diagnostische Testelemente 110 in Form von Teststreifen mit Plasma mit 100 mg/dl Glucose getüpfelt, und die Reaktionsfarbe wird mit einer CCD-Kamera vermessen. Da die einzelnen Pixel getrennt ausgelesen werden können, werden eine Anzahl an Pixeln statistisch bezüglich ihrer Präzision (d.h. bezüglich ihrer Standardabweichung) ausgewertet. Hierbei werden bei der höchsten Auflösung 10 Pixel (Kantenlänge 10 Mikrometer) ausgewertet, d.h. insgesamt eine Fläche von 1000 µm². Bei geringeren Auflösungen, d.h. größeren Flächen, wird über mehr Pixel gemittelt.

Wiederum werden hierbei verschiedene Proben untersucht, in Analogie zu den obigen Proben:
- Probe A'':: wie Probe A oben, ungemahlene Füllstoffe, Vergleichsprobe
- Probe J:: wie Probe A", jedoch Transpafill® und Fällungskieselsäure gemahlen,
- Probe A"':: wie Probe A oben, grobe Inhaltsstoffe, Vergleichsprobe, und
- Probe K:: Austausch von Transpafill® durch Aeroxide TiO₂ P 25.

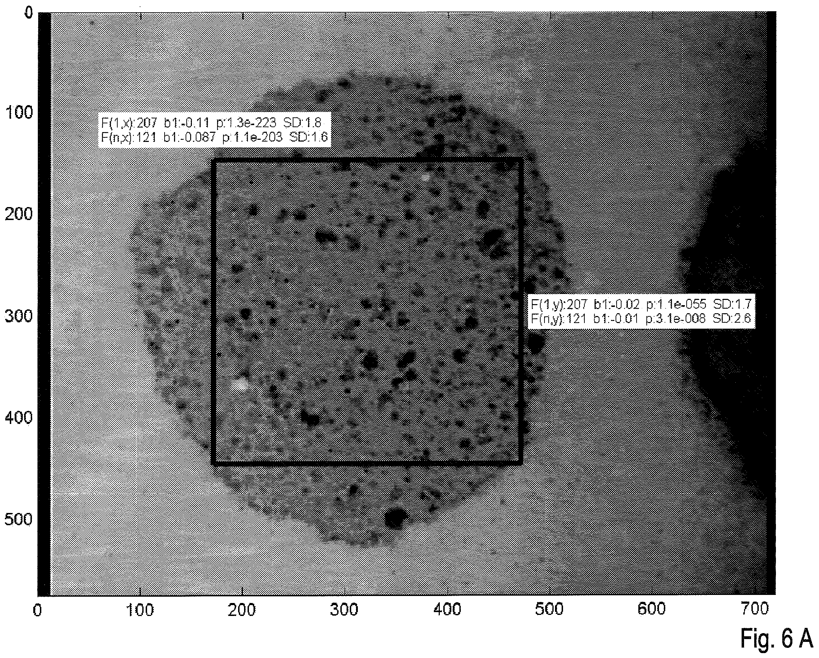

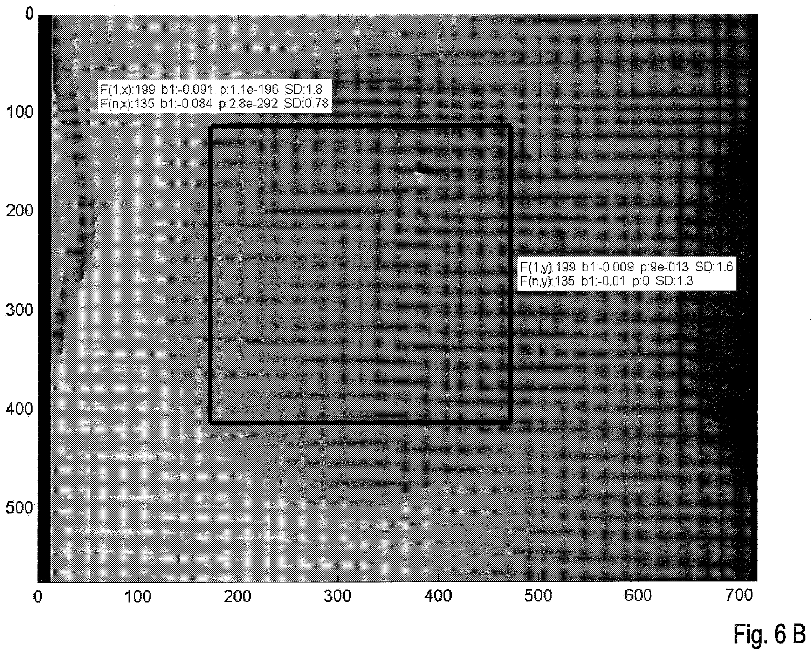

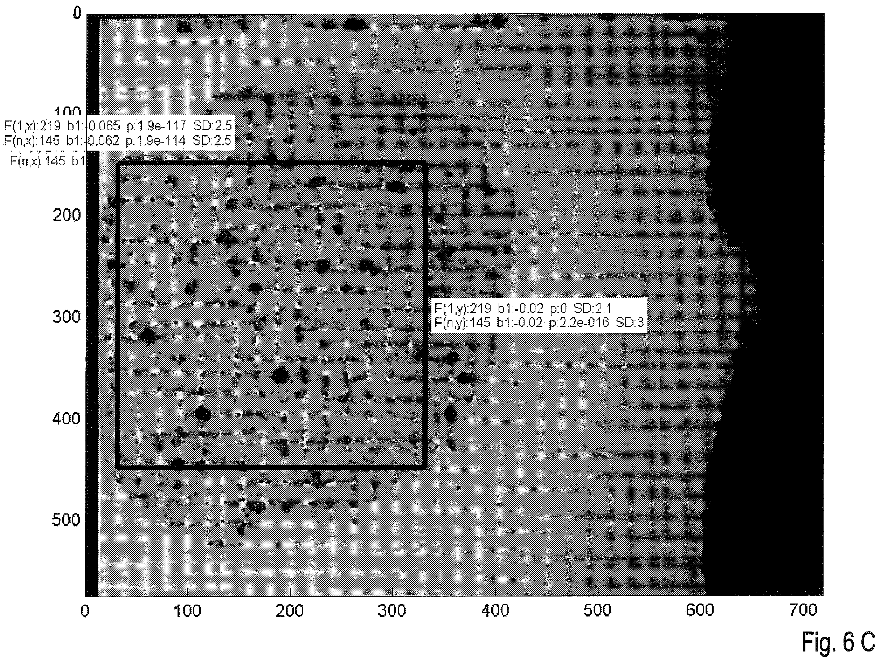

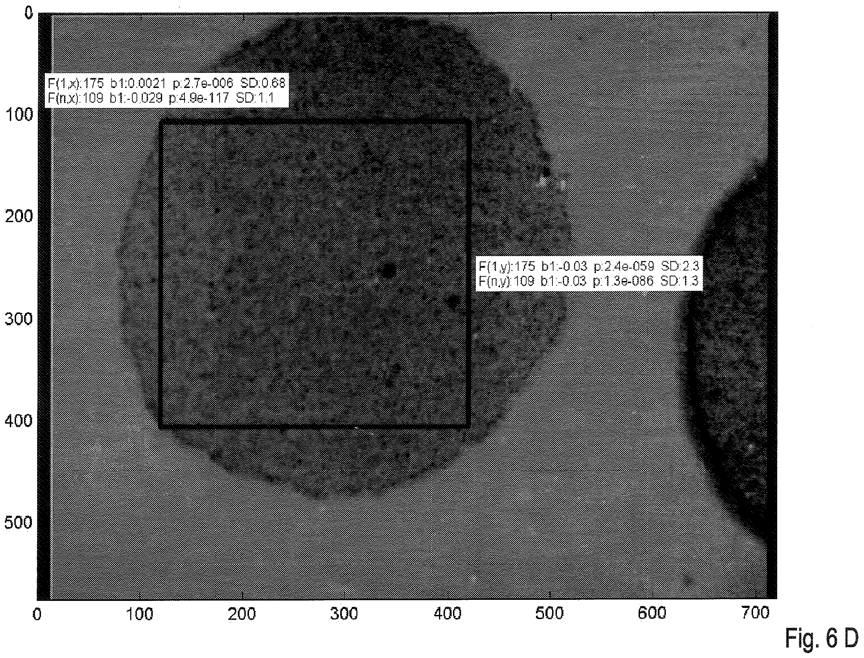

In den Figuren 6A bis 6C sind Mikroskopaufnahmen der Messflecken gezeigt, welche die Basis für die nachfolgenden Messungen darstellen. Dabei zeigt Figur 6A eine Mikroskopaufnahme eines mit der Lösung betüpfelten Bereichs der Probe A", also einer Probe mit ungemahlenen Füllstoffen. Figur 6B zeigt ein analoges Bild für die Probe J, also einer Probe mit gemahlener Testchemie. Figur 6C zeigt eine Aufnahme für die Probe A''', welche im Wesentlichen wiederum eine Probe mit groben Inhaltsstoffen darstellt und der Probe A" entspricht, und Figur 6D zeigt ein analoges Bild für die Probe K, in welcher Transpafill® durch Aeroxide TiO₂ P 25 ausgetauscht ist.

Die Achsenbeschriftungen in den Figuren 6A bis 6D geben jeweils in willkürlichen Einheiten die Pixelposition auf einem CCD-Chip wieder. Weiterhin sind in den Figuren 6A bis 6C Messfelder mittels entsprechender Quadrate markiert, deren Koordinaten in den Bildern wiedergegeben sind.

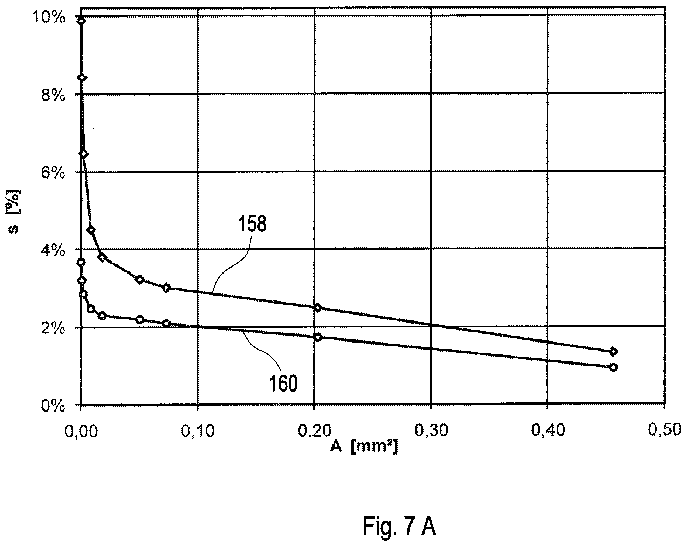

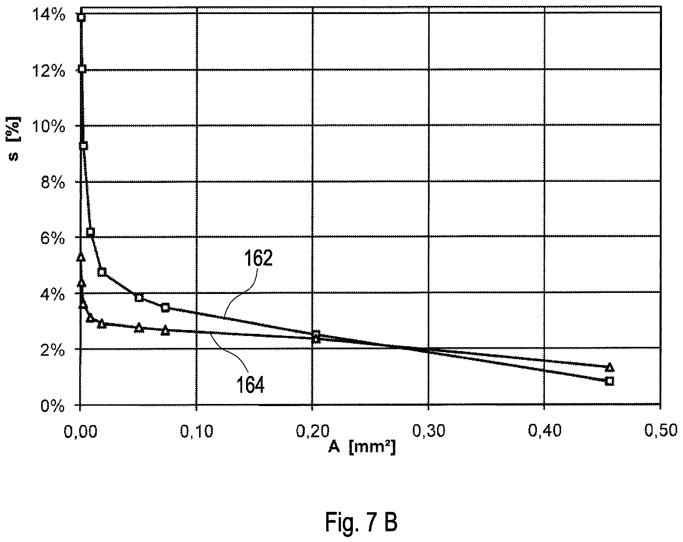

In den Figuren 7A und 7B sind jeweils Standardabweichungen für die Proben der Figuren 6A bis 6D dargestellt. Aufgetragen ist jeweils auf der vertikalen Achse die Standardabweichung s der Grauwerte in den Figuren 6A bis 6D. Diese Standardabweichung s ist angegeben in Prozent, bezogen auf den mittleren Grauwerthub zwischen einer Lehrmessung und einer ausreagierten Probe. Diese Standardabweichung s ist angegeben als Funktion der auf der horizontalen Achse aufgetragenen Fläche A, über welche gemittelt wurde.

Dabei zeigt 7A einen Vergleich der Proben A" und J, also einen Vergleich der Standard-probe mit einer Probe mit gemahlener Testchemie. Die Kurve 158 gibt den Verlauf der Standardabweichung für die Probe A" wieder, wohingegen die Bezugsziffer 160 die Kurve der Probe J mit gemahlener Testchemie bezeichnet. In Figur 7B ist die Standard-Probe A'" (Bezugsziffer 162) in ihrer Standardabweichung der Probe K (Bezugsziffer 164) gegenüber gestellt.

Die Messergebnisse zeigen, dass unterhalb ca. 30 x 30 µm², d.h. bei einer Fläche < 0,01 mm², die Standardabweichung s und damit der mögliche Messfehler stark zunehmen. Ferner ist zu erkennen, dass dieser Anstieg der Standardabweichung bei der Probe J (Kurve 160) mit der gemahlenen Chemie deutlich geringer ist, so dass für die Miniaturisierung der Blutvolumina, d.h. für eine Miniaturisierung des Messflecks in den Figuren 6A bis 6D, die gemahlene Chemie vorteilhaft ist. Ähnliches ergibt sich auch für die Probe K (Kurve 164 in Figur 7B).

Oben wurden verschiedene Verfahren angegeben, um die Partikelgröße von Proben zu bestimmen. Eine weitere, alternativ oder zusätzlich anwendbare Möglichkeit besteht darin, in Mikroskopaufnahmen wie beispielsweise gemäß den Figuren 6A bis 6D, Autokorrelationsfunktionen über die Grauwertverteilung zu berechnen. Die Autokorrelationsfunktion ist eine Kreuzkorrelationsfunktion eines Signals mit sich selbst, welche eine Funktion einer Verschiebung τ ist.

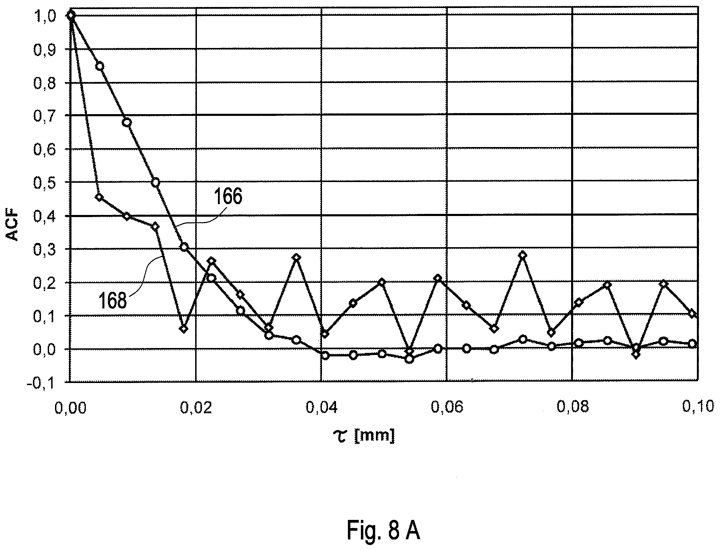

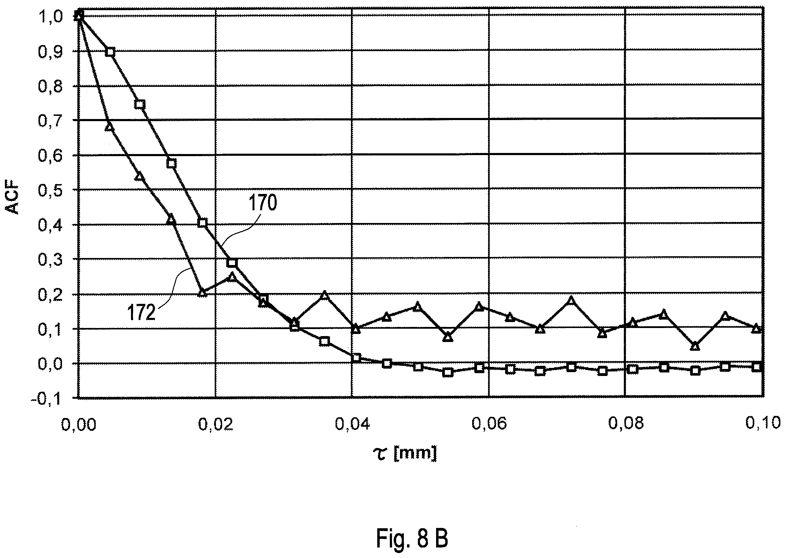

In den Figuren 8A und 8B sind Autokorrelationsfunktionen (dort bezeichnet mit ACF) für die Proben A" bis K aufgetragen. Dabei zeigt Figur 8A eine Gegenüberstellung der Vergleichsprobe A" (Kurve 166) und der Probe J mit gemahlenen Inhaltsstoffen (Kurve 168), und Figur 8B zeigt eine Gegenüberstellung der Vergleichsprobe A'" mit groben Inhaltsstoffen (Kurve 170) und der Probe K mit feinen Inhaltsstoffen (Kurve 172). Dargestellt ist jeweils auf der vertikalen Achse die Autokorrelationsfunktion ACF, und auf der horizontalen Achse die Verschiebung τ der Autokorrelationsfunktion in Millimetern. Die Autokorrelationsfunktionen wurden dabei durch eine Auswertung der Figuren 6A bis 6D ermittelt.

Durch Vergleich der Autokorrelationsfunktionen ist erkennbar, dass die groben Füllstoffe (Kurven 166, 170) deutlich breitere Autokorrelationsfunktionen aufweisen als die feinkörnigen Füllstoffe (Kurven 168, 172). Diese Darstellungen zeigen, dass die Autokorrelationsfunktion ACF mit der Partikelgrößenverteilung korreliert. Allein aus Mirkoaufnahmen, wie beispielsweise den Aufnahmen in den Figuren 6A bis 6D ist es somit möglich, die Körnigkeit einer Probe zu bestimmen. Beispielsweise kann die Halbwertsbreite der Autokorrelationsfunktionen 166 bis 172 ein Maß für die Körnigkeit der Nachweisschicht 118 sein. Zwar ist ein unmittelbares Ablesen der Partikelgrößenverteilung aus diesen Kurven 166 bis 172 nicht möglich. Mittels einer oder mehrerer Eichmessungen an Proben mit bekannten Partikelgrößenverteilungen kann aus den Kurven 166 bis 172 direkt auf die Partikelgröße bzw. die Partikelgrößenverteilung geschlossen werden.

### Bezugszeichenliste

- 110: Diagnostisches Testelement
- 112: Trägerelement
- 114: Transparenter Bereich
- 116: Testfeld
- 118: Nachweisschicht
- 119: Trägerfolie
- 120: Nachweisseite
- 122: Abtrennschicht
- 124: Testfeldoberfläche
- 126: Probe
- 128: Aufgabenseite
- 130: Tropfen
- 132: Teilbild Mikroskopaufnahme
- 134: Teilbild Grauwertänderung
- 136: Schnittlinie
- 137: Partikel
- 138: Remission Probe A
- 140: Remission Probe B
- 142: Remission Probe A
- 144: Remission Probe D
- 146: Remission Probe E
- 148: Remission Probe F
- 150: Remission Probe A'
- 152: Remission Probe G
- 154: Remission Probe H
- 156: Remission Probe I
- 158: Standardabweichung Probe A"
- 160: Standardabweichung Probe J
- 162: Standardabweichung Probe A'"
- 164: Standardabweichung Probe K
- 166: Autokorrelation Probe A''
- 168: Autokorrelation Probe J
- 170: Autokorrelation Probe A'''
- 172: Autokorrelation Probe K

## Patentansprüche

1. Diagnostisches Testelement (110) zum Nachweis eines Analyten in einer Probe (126) einer Körperflüssigkeit, insbesondere in Vollblut, umfassend mindestens ein Testfeld (116) mit mindestens einem Nachweisreagens, wobei das Nachweisreagens eingerichtet ist, um bei Anwesenheit des Analyten mindestens eine nachweisbare Veränderung zu durchlaufen, insbesondere eine optische Veränderung, wobei das Testfeld (116) mindestens eine das Nachweisreagens umfassende Nachweisschicht (118) aufweist, wobei die Nachweisschicht (118) Partikel (137) aufweist, wobei mindestens 90% sämtlicher Partikel (137) der Nachweisschicht (118) eine tatsächliche Partikelgröße von weniger als 10 Mikrometern aufweisen, wobei zur Bestimmung der Partikelgrößenverteilung ein elektronenmikroskopisches Verfahren eingesetzt wird, wobei nur Partikel ab einer Mindestgröße von 200 Nanometern bei der Bestimmung der Partikelgrößenverteilung berücksichtigt werden, wobei das Testfeld (116) eine Aufgabenseite (128) zum Aufbringen der Probe (126) und eine Nachweisseite (120) zum Nachweisen einer Veränderung des Nachweisreagens, insbesondere einer optischen Veränderung, aufweist, wobei das Testfeld (116) weiterhin mindestens eine Abtrennschicht (122) aufweist, wobei die Abtrennschicht (122) auf einer der Aufgabenseite (128) zuweisenden Seite der Nachweisschicht (118) angeordnet ist, wobei die Abtrennschicht (122) mindestens ein Pigment umfasst, dadurch charakterisiert, dass mindestens 70% sämtlicher Partikel (137) der Nachweisschicht (118) eine tatsächliche Partikelgröße von weniger als 900 Nanometern aufweisen.

2. Diagnostisches Testelement (110) nach dem vorhergehenden Anspruch, wobei mindestens 80% sämtlicher Partikel (137) der Nachweisschicht (118) eine tatsächliche Partikelgröße von weniger als 5 Mikrometern, insbesondere von weniger als 1 Mikrometer, aufweisen.

3. Diagnostisches Testelement (110) nach einem der vorhergehenden Ansprüche, wobei die Partikel (137) eines oder mehrere der folgenden Materialien umfassen: SiO₂; Kieselgur; ein Silikat, insbesondere ein Natrium-Aluminium-Silikat; ein Metalloxid, insbesondere ein Aluminiumoxid und/oder ein Titanoxid; ein synthetisches oxidisches Material, insbesondere ein nanoteiliges oxidisches Material, insbesondere ein nanoteiliges Siliziumoxid und/oder Aluminiumoxid und/oder Titanoxid; Kaolin; Pulverglas; Fällungskieselsäure; Calciumsulfat x 2 H₂O.

4. Diagnostisches Testelement (110) nach einem der vorhergehenden Ansprüche, wobei sämtliche Partikel (137) der Nachweisschicht (118) mit einer Partikelgröße von mehr als 100 nm anorganische Partikel (137) sind.

5. Diagnostisches Testelement (110) nach einem der vorhergehenden Ansprüche, wobei die Nachweisschicht (118) einen Brechungsindex zwischen 1,0 und 1,5, vorzugsweise zwischen 1,2 und 1,4, aufweist.

6. Diagnostisches Testelement (110) nach einem der vorhergehenden Ansprüche, wobei das Pigment ein weißes Pigment umfasst, vorzugsweise ein Pigment ausgewählt aus einem oder mehreren der folgenden Pigmente: Titandioxid; Zirkondioxid; Bariumtitanat; Bariumzirkonat; Zirkoniumsilikat.

7. Diagnostisches Testelement (110) nach einem der vorhergehenden Ansprüche, weiterhin umfassend mindestens ein Trägerelement (112), wobei das Trägerelement (112) mindestens einen transparenten Bereich (114) aufweist, wobei das Testfeld (116) mit seiner Nachweisseite (120) zumindest teilweise auf den transparenten Bereich (114) aufgebracht ist.

8. Verfahren zur Herstellung eines diagnostischen Testelements (110) zum Nachweis eines Analyten in einer Probe (126) einer Körperflüssigkeit, insbesondere eines diagnostischen Testelements (110) nach einem der vorhergehenden Ansprüche, wobei das diagnostische Testelement (110) mindestens ein Testfeld (116) mit mindestens einem Nachweisreagens umfasst, wobei das Nachweisreagens eingerichtet ist, um bei Anwesenheit des Analyten mindestens eine Veränderung zu durchlaufen, insbesondere eine optische Veränderung, wobei das Testfeld (116) mindestens eine das Nachweisreagens umfassende Nachweisschicht (118) aufweist, wobei die Nachweisschicht (118) derart erzeugt wird, dass die Nachweisschicht (118) Partikel (137) aufweist, wobei mindestens 90% sämtlicher Partikel (137) der Nachweisschicht (118) eine tatsächliche Partikelgröße von weniger als 10 Mikrometern, vorzugsweise von weniger als 1 Mikrometer, aufweisen, wobei zur Bestimmung der Partikelgrößenverteilung ein elektronenmikroskopisches Verfahren eingesetzt wird, wobei nur Partikel ab einer Mindestgröße von 200 Nanometern bei der Bestimmung der Partikelgrößenverteilung berücksichtigt werden, wobei das Testfeld (116) eine Aufgabenseite (128) zum Aufbringen der Probe (126) und eine Nachweisseite (120) zum Nachweisen einer Veränderung des Nachweisreagens, insbesondere einer optischen Veränderung, aufweist, wobei das Testfeld (116) weiterhin mindestens eine Abtrennschicht (122) aufweist, wobei die Abtrennschicht (122) auf einer der Aufgabenseite (128) zuweisenden Seite der Nachweisschicht (118) angeordnet ist, wobei die Abtrennschicht (122) mindestens ein Pigment umfasst, dadurch charakterisiert, dass mindestens 70% sämtlicher Partikel (137) der Nachweisschicht (118) eine tatsächliche Partikelgröße von weniger als 900 Nanometern aufweisen.

9. Verfahren nach dem vorhergehenden Anspruch, wobei zum Bereitstellen der Partikel (137) mindestens ein Pulver verwendet wird, wobei das Pulver Agglomerate von Primärteilchen aufweist, wobei das Pulver mittels mindestens eines mechanischen Dispergierverfahrens bearbeitet wird, um die Agglomerate zumindest teilweise aufzubrechen.

10. Verfahren nach dem vorhergehenden Anspruch, wobei zur Durchführung des mechanischen Dispergierverfahrens mindestens ein Dissolver verwendet wird, wobei eine Dispersion zur Herstellung der Nachweisschicht (118) erzeugt wird.

11. Verfahren nach einem der vorhergehenden Verfahrensansprüche, wobei zum Bereitstellen der Partikel (137) mindestens ein Pulver verwendet wird, wobei das Pulver mindestens einem Mahlschritt unterworfen wird.

12. Verfahren nach einem der vorhergehenden Verfahrensansprüche, wobei beim Bereitstellen der Partikel (137) ein Pulver eines synthetischen oxidischen Materials verwendet wird, insbesondere eines nanoteiligen oxidischen Materials, insbesondere ein nanoteiliges Siliziumoxid und/oder Aluminiumoxid und/oder Titanoxid.

13. Verfahren zum Nachweis eines Analyten in einer Probe (126) einer Körperflüssigkeit, insbesondere in Vollblut, wobei ein diagnostisches Testelement (110) nach einem der vorhergehenden, ein diagnostisches Testelement (110) betreffenden Ansprüche verwendet wird, wobei die Probe (126) ein Volumen von weniger als 2 Mikrolitern aufweist, insbesondere von weniger als 0,5 Mikrolitern.

14. Verfahren nach dem vorhergehenden Anspruch, wobei die nachweisbare Veränderung eine optisch nachweisbare Veränderung ist, wobei zum Nachweis der nachweisbaren Veränderung ein ortsauflösender optischer Detektor verwendet wird.

## Claims

1. Diagnostic test element (110) for detecting an analyte in a sample (126) of a body fluid, more particularly in whole blood, comprising at least one test field (116) having at least one detection reagent, wherein the detection reagent is set up to pass through at least one detectable change in the presence of the analyte, more particularly an optical change, wherein the test field (116) has at least one detection layer (118) comprising the detection reagent, wherein the detection layer (118) comprises particles (137), wherein at least 90% of all particles (137) of the detection layer (118) have an actual particle size of less than 10 micrometers, wherein for determining the particle size distribution an electron microscopy process is used, wherein only particles above a minimum size of 200 nanometers are considered in the determination of the particle size distribution, wherein the test field (116) has a loading side (128) for applying the sample (126) and a detection side (120) for detecting a change in the detection reagent, more particularly an optical change, wherein the test field (116) further has at least one partition layer (122), wherein the partition layer (122) is arranged on a side of the detection layer (118) facing the loading side (128), wherein the partition layer (122) comprises at least one pigment, **characterized in that** at least 70% of all particles (137) of the detection layer (118) have an actual particle size of less than 900 nanometers.

2. Diagnostic test element (110) according to the preceding claim, wherein at least 80% of all particles (137) of the detection layer (118) have an actual particle size of less than 5 micrometers, more particularly of less than 1 micrometer.

3. Diagnostic test element (110) according to either of the preceding claims, wherein the particles (137) comprise one or more of the following materials: SiO2; diatomaceous earth; a silicate, more particularly a sodium aluminum silicate; a metal oxide, more particularly an aluminum oxide and/or a titanium oxide; a synthetic oxidic material, more particularly a nanoparticulate oxidic material, more particularly a nanoparticulate silicon oxide and/or aluminum oxide and/or titanium oxide; kaolin; powder glass; precipitated silica; calcium sulfate x 2 H₂O.

4. Diagnostic test element (110) according to any of the preceding claims, wherein all particles (137) of the detection layer (118) having a particle size of more than 100 nm are inorganic particles (137).

5. Diagnostic test element (110) according to any of the preceding claims, wherein the detection layer (118) has a refractive index of between 1.0 and 1.5, preferably of between 1.2 and 1.4.

6. Diagnostic test element (110) according to any of the preceding claims, wherein the pigment comprises a white pigment, preferably a pigment selected from one or more of the following pigments: titanium dioxide; zirconium dioxide; barium titanate; barium zirconate; zirconium silicate.

7. Diagnostic test element (110) according to any of the preceding claims, further comprising at least one support element (112), wherein the support element (112) has at least one transparent region (114), wherein the test field (116) is applied at least in part to the transparent region (114) with its detection side (120).

8. Process for producing a diagnostic test element (110) for detecting an analyte in a sample (126) of a body fluid, more particularly a diagnostic test element (110) according to any of the preceding claims, wherein the diagnostic test element (110) comprises at least one test field (116) having at least one detection reagent, wherein the detection reagent is set up to pass through at least one change in the presence of the analyte, more particularly an optical change, wherein the test field (116) has at least one detection layer (118) comprising the detection reagent, wherein the detection layer (118) is generated such that the detection layer (118) comprises particles (137), wherein at least 90% of all particles (137) of the detection layer (118) have an actual particle size of less than 10 micrometers, preferably of less than 1 micrometer, wherein for determining the particle size distribution an electron microscopy process is used, wherein only particles above a minimum size of 200 nanometers are considered in the determination of the particle size distribution, wherein the test field (116) has a loading side (128) for applying the sample (126) and a detection side (120) for detecting a change in the detection reagent, more particularly an optical change, wherein the test field (116) further has at least one partition layer (122), wherein the partition layer (122) is arranged on a side of the detection layer (118) facing the loading side (128), wherein the partition layer (122) comprises at least one pigment, **characterized in that** at least 70% of all particles (137) of the detection layer (118) have an actual particle size of less than 900 nanometers.

9. Process according to the preceding claim, wherein at least one powder is used to provide the particles (137), wherein the powder comprises agglomerates of primary particulates, wherein the powder is processed by means of at least one mechanical dispersion process in order to break up the agglomerates at least partly.

10. Process according to the preceding claim, wherein at least one dissolver is used for carrying out the mechanical dispersion process, wherein a dispersion for producing the detection layer (118) is generated.

11. Process according to any of the preceding process claims, wherein at least one powder is used to provide the particles (137), wherein the powder is subjected to at least one milling step.

12. Process according to any of the preceding process claims, wherein a powder of a synthetic oxidic material is used when providing the particles (137), more particularly a nanoparticulate oxidic material, more particularly a nanoparticulate silicon oxide and/or aluminum oxide and/or titanium oxide.

13. Process for detecting an analyte in a sample (126) of a body fluid, more particularly in whole blood, wherein a diagnostic test element (110) according to any of the preceding claims relating to a diagnostic test element (110) is used, wherein the sample (126) has a volume of less than 2 microliters, more particularly of less than 0.5 microliters.

14. Process according to the preceding claim, wherein the detectable change is an optically detectable change, wherein a spatially resolving optical detector is used for detecting the detectable change.

## Revendications

1. Élément de test de diagnostic (110) destiné à détecter un analyte dans un échantillon (126) d'un liquide corporel, en particulier dans le sang complet, comprenant au moins une zone de test (116) qui comprend au moins un réactif de détection, le réactif de détection étant conçu pour, en présence de l'analyte, subir au moins une modification détectable, en particulier une modification optique, la zone de test (116) comprenant au moins une couche de détection (118) qui comprend le réactif de détection, la couche de détection (118) comprenant des particules (137), au moins 90 % de toutes les particules (137) de la couche de détection (118) ayant une taille de particule effective inférieure à 10 micromètres, un procédé de microscopie électronique étant utilisé pour déterminer la distribution des tailles de particules, seules les particule à partir d'une taille minimale de 200 nanomètres étant prises en compte lors de la détermination de la distribution des tailles de particules, la zone de test (116) comprenant un côté de chargement (128) pour l'application de l'échantillon (126) et un côté de détection (120) pour la détection d'une modification du réactif de détection, en particulier d'une modification optique, la zone de test (116) comprenant en outre au moins une couche de séparation (122), la couche de séparation (122) étant agencée sur un côté de la couche de détection (118) orienté vers le côté de chargement (128), la couche de séparation (122) comprenant au moins un pigment, **caractérisé en ce qu'**au moins 70 % de toutes les particules (137) de la couche de détection (118) présentent une taille de particule effective inférieure à 900 nanomètres.

2. Élément de test de diagnostic (110) selon la revendication précédente, dans lequel au moins 80 % de toutes les particules (137) de la couche de détection (118) présentent une taille de particule effective inférieure à 5 micromètres, en particulier inférieure à 1 micromètre.

3. Élément de test de diagnostic (110) selon l'une quelconque des revendications précédentes, dans lequel les particules (137) comprennent un ou plusieurs des matériaux suivants : SiO₂ ; la diatomite ; un silicate, en particulier un silicate de sodium et d'aluminium ; un oxyde métallique, en particulier un oxyde d'aluminium et/ou un oxyde de titane ; un matériau oxydique synthétique, en particulier un matériau oxydique nanoparticulaire, en particulier un oxyde de silicium et/ou un oxyde d'aluminium et/ou un oxyde de titane nanoparticulaire ; le kaolin ; le verre en poudre ; la silice précipitée ; le sulfate de calcium x 2 H₂O.

4. Élément de test de diagnostic (110) selon l'une quelconque des revendications précédentes, dans lequel toutes les particules (137) de la couche de détection (118) ayant une taille de particule supérieure à 100 nm sont des particules inorganiques (137).

5. Élément de test de diagnostic (110) selon l'une quelconque des revendications précédentes, dans lequel la couche de détection (118) présente un indice de réfraction compris entre 1,0 et 1,5, de préférence entre 1,2 et 1,4.

6. Élément de test de diagnostic (110) selon l'une quelconque des revendications précédentes, dans lequel le pigment comprend un pigment blanc, de préférence un pigment choisi parmi un ou plusieurs des pigments suivants : le dioxyde de titane, le dioxyde de zirconium, le titanate de baryum, le zirconate de baryum, le silicate de zirconium.

7. Élément de test de diagnostic (110) selon l'une quelconque des revendications précédentes, comprenant en outre au moins un élément support (112), l'élément support (112) comprenant au moins une zone transparente (114), la zone de test (116) étant au moins partiellement appliquée avec son côté de détection (120) sur la zone transparente (114).

8. Procédé de fabrication d'un élément de test de diagnostic (110) destiné à détecter un analyte dans un échantillon (126) d'un liquide corporel, en particulier d'un élément de test de diagnostic (110) selon l'une quelconque des revendications précédentes, l'élément de test de diagnostic (110) comprenant au moins une zone de test (116) qui comprend au moins un réactif de détection, le réactif de détection étant conçu pour, en présence de l'analyte, subir au moins une modification, en particulier une modification optique, la zone de test (116) comprenant au moins une couche de détection (118) qui comprend le réactif de détection, la couche de détection (118) étant formée de telle sorte que la couche de détection (118) comprenne des particules (137), au moins 90 % de toutes les particules (137) de la couche de détection (118) ayant une taille de particule effective inférieure à 10 micromètres, de préférence inférieure à 1 micromètre, un procédé de microscopie électronique étant utilisé pour déterminer la distribution des tailles de particules, seules les particule à partir d'une taille minimale de 200 nanomètres étant prises en compte lors de la détermination de la distribution des tailles de particules, la zone de test (116) comprenant un côté de chargement (128) pour l'application de l'échantillon (126) et un côté de détection (120) pour la détection d'une modification du réactif de détection, en particulier d'une modification optique, la zone de test (116) comprenant en outre au moins une couche de séparation (122), la couche de séparation (122) étant agencée sur un côté de la couche de détection (118) orienté vers le côté de chargement (128), la couche de séparation (122) comprenant au moins un pigment, **caractérisé en ce qu'**au moins 70 % de toutes les particules (137) de la couche de détection (118) présentent une taille de particule effective inférieure à 900 nanomètres.

9. Procédé selon la revendication précédente, dans lequel au moins une poudre est utilisée pour la préparation des particules (137), la poudre comprenant des agglomérats de particules primaires, la poudre étant traitée au moyen d'au moins un procédé de dispersion mécanique afin de fragmenter au moins partiellement les agglomérats.

10. Procédé selon la revendication précédente, dans lequel au moins un dissolveur est utilisé pour la réalisation du procédé de dispersion mécanique, une dispersion étant formée pour la fabrication de la couche de détection (118).

11. Procédé selon l'une quelconque des revendications de procédé précédentes, dans lequel au moins une poudre est utilisée pour la préparation des particules (137), la poudre étant soumise à au moins une étape de broyage.

12. Procédé selon l'une quelconque des revendications de procédé précédentes, dans lequel une poudre d'un matériau oxydique synthétique est utilisée lors de la préparation des particules (137), en particulier un matériau oxydique nanoparticulaire, en particulier un oxyde de silicium et/ou un oxyde d'aluminium et/ou un oxyde de titane nanoparticulaire.

13. Procédé de détection d'un analyte dans un échantillon (126) d'un liquide corporel, en particulier dans le sang complet, selon lequel un élément de test de diagnostic (110) selon l'une quelconque des revendications précédentes concernant un élément de test de diagnostic (110) est utilisé, l'échantillon (126) présentant un volume inférieur à 2 microlitres, en particulier inférieur à 0,5 microlitre.

14. Procédé selon la revendication précédente, dans lequel la modification détectable est une modification détectable optiquement, un détecteur optique à résolution spatiale étant utilisé pour la détection de la modification détectable.
